# EUROPEAN PATENT APPLICATION

(11) **EP 4 772 523 A1**
(43) Date of publication of application: **08.07.2026**
(21) Application number: 24859759.3
(22) Date of filing: 27.08.2024
(51) Int. Cl.: C07K 7/00, A61K 38/08, A61K 47/64, A61K 49/00, A61K 49/04, A61K 49/14, A61K 51/08, A61P 35/00, C12Q 1/02

(54) **D-PEPTIDE AND USE THEREOF**

(30) Priority: 28.08.2023 JP 2023137833
(71) Applicant: HIROSAKI UNIVERSITY, Hirosaki-shi, Aomori 036-8560 (JP)
(72) Inventor: YONEYAMA, Tohru, Hirosaki-shi, Aomori 036-8560 (JP); OHYAMA, Chikara, Hirosaki-shi, Aomori 036-8560 (JP); HATAKEYAMA, Shingo, Hirosaki-shi, Aomori 036-8560 (JP); NONAKA, Motohiro, Kyoto-shi, Kyoto 606-8397 (JP)
(74) Representative: Regimbeau
(86) International application number: PCT/JP2024/030462
(87) International publication number: WO 2025/047727

(57) **Abstract**

The present invention provides a peptide comprising an amino acid sequence represented by any one of the formulas (I) to (V) (wherein each amino acid symbol immediately following the symbol [D] represents the D-form of the corresponding amino acid): (I) [D](X1)[D](X2)[D](X3)[D]E[D]V[D]R[D]S, wherein X1 represents H or Q, X2 represents P or S, and X3 represents N or K, (II) [D]Q[D](X2)[D]A[D]T[D](X5)[D]L[D]K, wherein X2 represents Y or L, and X5 represents N, K, or Y, (III) [D](X1)[D]T[D]S[D](X4)[D](X5)[D]T[D]L, wherein X1 represents S or R, X4 represents R or W, and X5 represents N or I, (IV) any one of the amino acid sequences (I) to (III), which has one or several amino acid insertions, one or several amino acid substitutions, or one or several amino acid deletions, or a combination thereof, and (V) an amino acid sequence that is a retro-inverso of any one of the amino acid sequences (I) to (IV).

## Description

### TECHNICAL FIELD

The present invention relates to a peptide that includes specific amino acids and binds to annexin A1, and a conjugate including the peptide and one or more components. The present invention also relates to a composition including the peptide or conjugate, use thereof, and the like.

### BACKGROUND ART

In recent years, in the field of anticancer agent treatment, antibody-drug conjugates (ADCs) have attracted attention, in which an anticancer agent having a high cell-killing effect but having strong side effects is conjugated to an antibody targeting a molecule on the cancer cell surface. ADCs are compounds in which a small molecule having a high cell-killing effect is conjugated to an antibody so as to be capable of being released at the target site, and are expected as next-generation antibody medicines. The ADCs can selectively deliver the conjugated small molecule to the target site, because the antibody binds with high affinity to the antigen present at the target site. Therefore, characteristics of the antibody and the low-molecular anticancer agent can be utilized at the same time. That is, the antibody has extremely high affinity for the antigen and has a long half-life, but is able to target only the antigen present on the cell surface. On the other hand, the low-molecular anticancer agent has low selectivity and a short in-vivo retention time, but has high permeability and is able to target the intracellular protein as well. The ADCs are extremely suitable as DDS (drug delivery system) formulations, in which the small molecule is conjugated to the antibody having a long half-life to release the low-molecular anticancer agent at the target site in a sustained manner and target the intracellular protein.

However, with the rising cost of research and development of antibody pharmaceuticals based on the ADCs, patients are now facing greater financial burdens. There is a concern that this trend may be accelerated due to advanced medical care such as diagnostic systems utilizing genetic information in the future. Such rising drug prices will also have an immeasurable effect on the national medical expenditure. Further, expensive antibody pharmaceuticals will not be affordable for people in developing countries and will widen inequalities in healthcare across the world. As measures for such problems in the longer term, it is necessary not only to search for excellent pharmaceutical seeds, but also to explore the possibility of inexpensive biopharmaceuticals such as oligopeptides in the future.

Peptide-drug conjugates (PDCs) are known as a strategy similar to the ADCs described above. In recent years, PDCs have been reported to successfully inhibit glycan-dependent cancer metastasis for the first time in the world using a peptide mimicking the glycan structure (Non-Patent Literature 1). It has also been reported that a peptide named IF7 was found to bind to annexin A1 (ANXA1) during examination of vascular endothelial receptors with which such glycan-mimetic peptides interact (Non-Patent Literature 2). Similarly, a peptide named dTIT7 is known which can bind to ANXA1 and can be used in a PDC for a DDS for anticancer agents (Patent Literature 1).

ANXA1 was found to have high specificity among currently known tumor vessel-specific marker molecules. It is intracellularly expressed in normal cells, but is strongly expressed on the luminal surface in contact with the blood flow in tumor neovascular endothelial cells (Non-Patent Literature 3). It has also been reported that ANXA1 is highly expressed in multiple types of cancer and this is related to the prognosis (Non-Patent Literature 4).

Although the aforementioned IF7 and dTIT7 are known as peptides targeting ANXA1, cancer therapy by PDCs utilizing such peptides may not be sufficiently effective as intended, because the peptides are poorly soluble and have low in-vivo stability, for example. Therefore, development of peptides targeting ANXA1 and suitable for PDCs has been desired.

### Citation List

### Patent Literature

Patent Literature 1: WO 2018/034356

### Non-Patent Literature

Non-Patent Literature 1: Fukuda et al., Cancer Res., 60:450-6, 2000
Non-Patent Literature 2: Hatakeyama et al., Proc. Natl. Acad. Sci. USA, 108: 19587-92, 2011
Non-Patent Literature 3: Oh et al., Nature, 429: 629-35, 2004
Non-Patent Literature 4: Lauren K et al., Immunology, 166(1):2-16, 2022

### SUMMARY OF INVENTION

### Technical Problem

Accordingly, an object of the present invention is to provide a peptide that includes specific amino acids and binds to annexin A1, and a conjugate including the peptide and one or more components. Another object of the present invention is to provide a composition including the peptide or conjugate, use thereof, and the like.

### Solution to Problem

The present inventors have come up with a method of screening for only the D-peptide sequence that differs from dTIT7 and binds to the N-terminal 15-mer L-peptide sequence of annexin A1 within five minutes in vivo at 37°C. Thus, the inventors have performed mirror-image T7 phage library screening to attempt to identify the D-peptide selectively binding to the N-terminus of ANXA1. As a result, a novel peptide has been successfully obtained which has seven specific D-amino acid residues and has high affinity for ANXA1. The obtained peptide also exhibited high solubility as a superior characteristic. Further, it has surprisingly been found that the obtained peptide accumulates in a tumor-specific manner in a bladder cancer model, a prostate cancer model, and a head and neck cancer model for 24 hours after administration to mice. In view of such results, the present inventors have assumed that the obtained peptide is suitable for a DDS for boron compounds or anticancer agents.

The present inventors have previously confirmed that an IF7-¹⁰B compound in which a boron compound is conjugated to IF7 accumulates in tumor tissues and thus can exhibit a therapeutic effect in boron neutron capture therapy (BNCT) which is a recently approved heavy ion radiotherapy having a tumor-specific cytotoxic effect (WO 2019/244954; Yoneyama et al., BMC cancer, 21:72, 2021). However, IF7 is poorly soluble and has low blood stability, disadvantageously, and the inventors have searched for a novel peptide for PDCs as a fundamental solution. Therefore, the inventors have studied whether the obtained highly soluble peptide can be used for BNCT. As a result of extensive studies, the inventors have found that in BNCT utilizing the obtained peptide, a conjugate (a peptide-drug conjugate) in which a boron compound is conjugated to the peptide exhibits extremely high tumor accumulation and has an extremely high antitumor effect. As a result of further studies based on such findings, the inventors have completed the present invention relating to a peptide that includes specific amino acids and binds to annexin A1, and a conjugate including the peptide and one or more components. The inventors have also completed the present invention relating to a composition including the peptide or conjugate, use thereof (e.g., for neutron capture therapy, cancer chemotherapy, or cancer tests), and the like.

Specifically, the present invention is as follows:
[1] A peptide comprising an amino acid sequence represented by any one of the formulas (I) to (V) (wherein each amino acid symbol immediately following the symbol [D] represents the D-form of the corresponding amino acid):
   (I) [D](X1)[D](X2)[D](X3)[D]E[D]V[D]R[D]S, wherein X1 represents H or Q, X2 represents P or S, and X3 represents N or K,
   (II) [D]Q[D](X2)[D]A[D]T[D](X5)[D]L[D]K, wherein X2 represents Y or L, and X5 represents N, K, or Y,
   (III) [D](X1)[D]T[D]S[D](X4)[D](X5)[D]T[D]L, wherein X1 represents S or R, X4 represents R or W, and X5 represents N or I,
   (IV) any one of the amino acid sequences (I) to (III), which has one or several amino acid insertions, one or several amino acid substitutions, or one or several amino acid deletions, or a combination thereof, and
   (V) an amino acid sequence that is a retro-inverso of any one of the amino acid sequences (I) to (IV).
[2] The peptide according to [1], comprising any one of the amino acid sequences (I') to (V) (wherein the symbol [D] is as defined above):
   (I') [D](X1)[D](X2)[D]N[D]E[D]V[D]R[D]S, wherein X1 represents H or Q, and X2 represents P or S,
   (II) [D]Q[D](X2)[D]A[D]T[D](X5)[D]L[D]K, wherein X2 represents Y or L, and X5 represents N, K, or Y,
   (III) [D](X1)[D]T[D]S[D](X4)[D](X5)[D]T[D]L, wherein X1 represents S or R, X4 represents R or W, and X5 represents N or I,
   (IV) any one of the amino acid sequences (I') to (III), which has one or several amino acid insertions, one or several amino acid substitutions, or one or several amino acid deletions, or a combination thereof, and
   (V) an amino acid sequence that is a retro-inverso of any one of the amino acid sequences (I') to (IV).
[3] The peptide according to [1] or [2], comprising any one of the amino acid sequences (i) to (iii) (wherein the symbol [D] is as defined above):
   (i) [D]H[D]P[D]N[D]E[D]V[D]R[D]S,
   (ii) [D]S[D]T[D]S[D]R[D]N[D]T[D]L, and
   (iii) [D]Q[D]Y[D]A[D]T[D]N[D]L[D]K.
[4] The peptide according to any one of [1] to [3], comprising an amino acid sequence represented by the formula (i) (wherein the symbol [D] is as defined above):
   (i) [D]H[D]P[D]N[D]E[D]V[D]R[D]S.
[5] A conjugate comprising the peptide according to any one of [1] to [4] and one or more components.
[6] The conjugate according to [5], wherein the one or more components include a boron or gadolinium compound.
[7] The conjugate according to [6], wherein the one or more components include a boron compound.
[8] The conjugate according to [7], wherein the conjugate is represented by the formula (I): 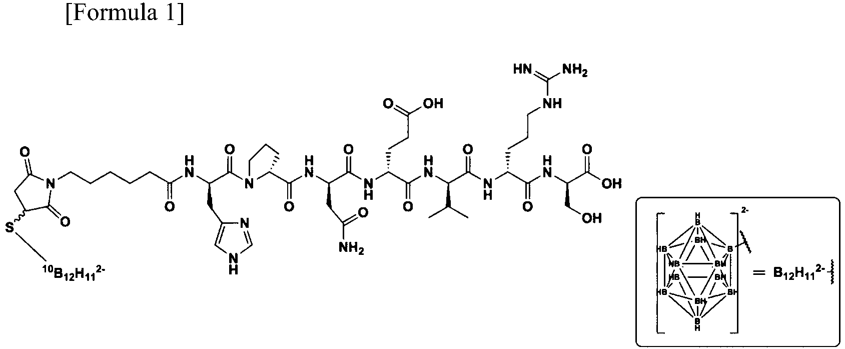 or the formula (II): 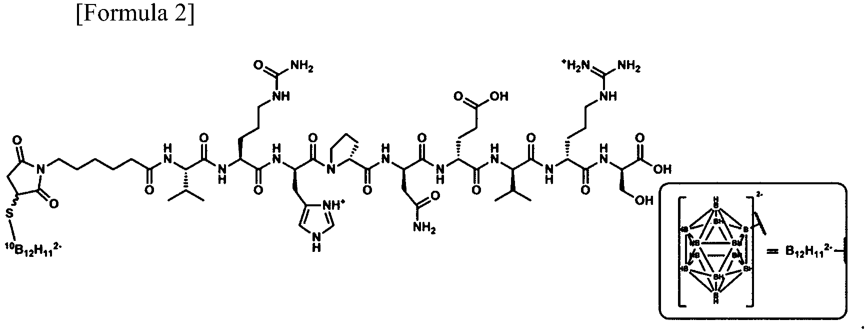
[9] The conjugate according to [5], wherein the one or more components include an anticancer agent.
[10] The conjugate according to [5], wherein the one or more components include a detectable substance.
[11] The conjugate according to [10], wherein the detectable substance allows the conjugate to be detected in vivo by a means selected from the group consisting of radiography, computed tomography (CT), magnetic resonance imaging (MRI), ultrasonography, scintigraphy, positron emission tomography (PET), radionuclide therapy, endoscopy, and laparoscopy.
[12] The conjugate according to claim [10] or [11], wherein the detectable substance is a radioisotope, an MRI contrast enhancer, a radiopaque substance, a contrast medium, or a fluorescent substance.
[13] A pharmaceutical composition comprising the peptide according to any one of [1] to [4] or the conjugate according to any one of [5] to [12].
[14] A neutron capture therapy agent comprising the conjugate according to any one of [6] to [8].
[15] The neutron capture therapy agent according to [14], wherein the conjugate includes a boron compound.
[16] The neutron capture therapy agent according to [14] or [15], for the treatment or prevention of solid cancer.
[17] The neutron capture therapy agent according to [16], wherein the solid cancer is selected from the group consisting of skin cancer, brain and central nervous system cancer, laryngeal cancer, oral cancer, salivary gland cancer, paranasal sinus cancer, thyroid cancer, bladder cancer, prostate cancer, cancer of the renal pelvis and ureter, and osteosarcoma.
[18] A cancer chemotherapeutic agent comprising the conjugate according to [9].
[19] A cancer testing reagent comprising the conjugate according to any one of [10] to [12].
[20] A neutron capture therapy comprising administering to a subject an effective dose of the conjugate according to any one of [6] to [8].
[21] The neutron capture therapy according to [20], wherein the subject suffers from solid cancer.
[22] The neutron capture therapy according to [21], wherein the solid cancer is selected from the group consisting of skin cancer, brain and central nervous system cancer, laryngeal cancer, oral cancer, salivary gland cancer, paranasal sinus cancer, thyroid cancer, bladder cancer, prostate cancer, cancer of the renal pelvis and ureter, and osteosarcoma.
[23] A method for treating or preventing cancer, comprising administering to a subject an effective dose of the conjugate according to any one of [6] to [9].
[24] A method for detecting cancer, comprising administering to a subject an effective dose of the conjugate according to any one of [10] to [12].
[25] The method according to [23] or [24], wherein the cancer is solid cancer.
[26] The neutron capture therapy agent according to [16] or the method according to [25], wherein the solid cancer is annexin A1-positive solid cancer.
[27] The neutron capture therapy agent according to any one of [14] to [17] or the cancer chemotherapeutic agent according to [18], for administering to a subject suffering from annexin A1-positive cancer who is selected from subjects suffering from cancer.
[28] A companion diagnostic agent for the neutron capture therapy agent according to any one of [14] to [17] or the cancer chemotherapeutic agent according to [18], the companion diagnostic agent comprising an annexin A1-binding molecule.
[29] A method for treating or preventing cancer, comprising selecting a subject suffering from cancer containing annexin A1-positive cells from subjects suffering from cancer; and administering the neutron capture therapy agent according to any one of [14] to [17] or the cancer chemotherapeutic agent according to [18] to the selected subject.
[30] The peptide according to any one of [1] to [4] or the conjugate according to any one of [5] to [12] for use in the treatment or prevention of cancer.
[31] Use of the peptide according to any one of [1] to [4] or the conjugate according to any one of [5] to [12] for the treatment or prevention of cancer.
[32] Use of the peptide according to any one of [1] to [4] or the conjugate according to any one of [5] to [12] in the manufacture of a cancer therapeutic or prophylactic agent.

### Advantageous Effects of Invention

According to the present invention, the obtained D-peptide can bind with high affinity to ANXA1 having high specificity among tumor vessel-specific marker molecules. The D-peptide is highly soluble as compared with conventionally known peptides, and is thus extremely useful for PDCs targeting ANXA1. The D-peptide accumulates at the tumor site continuously and remarkably, and can be expected to, for example, improve the efficiency of drug uptake in cancer cells and the antitumor effect in cancer such as bladder cancer, prostate cancer, or head and neck cancer, as compared with conventionally known peptides. Therefore, for example, the D-peptide is extremely useful for applications such as medical care (e.g., cancer therapy, more specifically, (boron) neutron capture therapy or cancer chemotherapy; or cancer tests, more specifically, monitoring of the therapeutic effect on a disease by in-vivo imaging). The D-peptide can also be expected to reduce side effects of the drug when it is used for a PDC.

### Brief Description of Drawings

[FIG. 1] FIG. 1 summarizes mirror-image phage display screening for identification of the L-amino acid peptide sequence binding to the N-terminal 15-mer peptide of annexin A1 consisting of a D-amino acid.
[FIG. 2] FIG. 2 summarizes the identification of the L-peptide sequence binding to D-MC16 by mirror-image phage display screening, for the 7-mer peptide sequences displayed by the phage mixtures obtained in the 1st to 5th outputs.
[FIG. 3] FIG. 3 shows the genome copy numbers of the phages eluted on respective rounds, for the 7-mer peptide sequences displayed by the phage mixtures obtained in the 1st to 5th outputs.
[FIG. 4] FIG. 4 shows the enrichment ratios of the L-amino acid sequences displayed by the phages eluted on respective rounds versus the 1st round pool, for the 7-mer peptide sequences displayed by the phage mixtures obtained in the 1st to 5th outputs.
[FIG. 5] FIG. 5 shows the result of alignment of the top 10 ranked peptide sequences, for the 7-mer peptide sequences displayed by the phage mixtures obtained in the 1st to 5th outputs.
[FIG. 6] FIG. 6 shows the KD and Rmax values of L-MC16-binding D-peptides versus L-MC16 or D-MC16. A: A sensorgram for association and dissociation and the equilibrium dissociation constant KD values of L-MC16 or D-MC16 peptides versus a dhp7-immobilized sensor. B: A sensorgram for association and dissociation and the equilibrium dissociation constant KD value of an L-MC16 peptide versus a dst7-immobilized sensor. C: A sensorgram for association and dissociation and the equilibrium dissociation constant KD value of an L-MC16 peptide versus a dqy7-immobilized sensor.

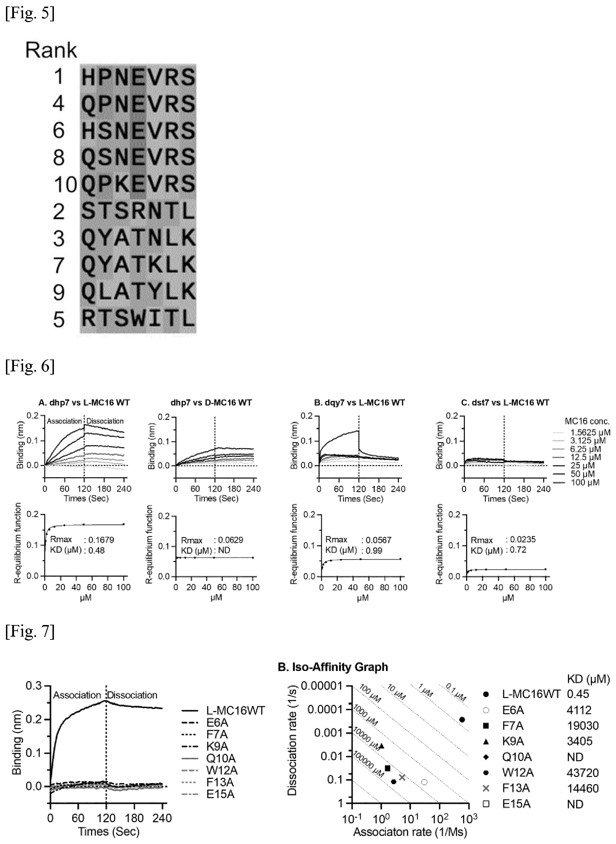

[FIG. 7] FIG. 7 shows the KD values and an isoaffinity graph of the dhp7 peptide versus L-MC16 mutants. A: A sensorgram for association and dissociation of L-MC16WT, E6A, F7A, K9A, Q10A, W11A, F13A, and E15A peptides versus a dhp7-immobilized sensor. B: An isoaffinity graph for association and dissociation and the equilibrium dissociation constant KD values of L-MC16WT, E6A, F7A, K9A, Q10A, W11A, F13A, and E15A peptides versus a dhp7-immobilized sensor. A two-dimensional isoaffinity kinetic plot of rate constants grouped by L-MC16 peptide series. The dashed diagonals represent equilibrium association constants and are shown to aid in visualization of the affinity distribution.
[FIG. 8] FIG. 8 relates to binding and intracellular uptake of the dhp7 peptide in HEK293 overexpressing Anxa1-c-His.
[FIG. 9] FIG. 9 shows the results of binding and intracellular uptake of the dhp7 peptide in HEK293 overexpressing Anxa1-c-His.
[FIG. 10] FIG. 10 shows the results of analysis of expression of ANXA1 on the cell surface of cancer cell lines by flow cytometry.
[FIG. 11] FIG. 11 shows the results of examination of tumor accumulation of Cy7.5-dhp7 in bladder cancer-bearing mice.
[FIG. 12] FIG. 12 shows the results of examination of tumor accumulation of Cy7.5-dhp7 in prostate cancer-bearing nude mice.
[FIG. 13] FIG. 13 shows the results of examination of tumor accumulation of Cy7.5-dhp7 in bladder cancer-bearing nude mice.
[FIG. 14] FIG. 14 shows the results of examination of tumor accumulation of Cy7.5-dhp7 in head and neck cancer-bearing nude mice.
[FIG. 15] FIG. 15 shows immunization with ¹⁰BSH-BSA and the results of analysis of affinity of rabbit anti-¹⁰BSH polyclonal antibodies (#52 and #53) for ¹⁰BSH-BSA.
[FIG. 16] FIG. 16 shows the results of visualization of uptake of the ¹⁰BSH-dhp7 peptide in ANXA 1-positive cancer cells by immunofluorescent staining.
[FIG. 17] FIG. 17 shows the results of neutron capture therapy after administration of ¹⁰BSH-dhp7 in cancer-bearing mice.
[FIG. 18] FIG. 18 shows the results of neutron capture therapy after administration of ¹⁰BSH-dhp7 in cancer-bearing mice.
[FIG. 19] FIG. 19 shows the results of immunostaining of resected tumors after neutron capture therapy following administration of ¹⁰BSH-dhp7.
[FIG. 20] FIG. 20 shows the results of examination of tumor accumulation of Cy7.5-vc-dhp7 in prostate cancer-bearing mice having a tumor size of 7 mm.
[FIG. 21] FIG. 21 shows the results of examination of tumor accumulation of Cy7.5-vc-dhp7 in prostate cancer-bearing mice having a tumor size of 7 mm.
[FIG. 22] FIG. 22 shows the results of examination of tumor accumulation of Cy7.5-vc-dhp7 in prostate cancer-bearing mice having a tumor size of 15 mm.
[FIG. 23] FIG. 23 shows the results of ex vivo examination of accumulation of Cy7.5-vc-dhp7 in each organ in prostate cancer-bearing mice, head and neck cancer-bearing mice, and osteosarcoma-bearing mice having a tumor size of 15 mm.
[FIG. 24] FIG. 24 shows the results of ex vivo examination of accumulation of Cy7.5-vc-dhp7 in each organ in prostate cancer-bearing mice having a tumor size of 15 mm.
[FIG. 25] FIG. 25 shows the results of examination of tumor accumulation of Cy7.5-vc-dhp7 in prostate cancer-bearing mice having a tumor size of 7 mm.
[FIG. 26] FIG. 26 shows the results of examination of tumor accumulation of Cy7.5-vc-dhp7 in head and neck cancer-bearing mice and osteosarcoma-bearing mice having a tumor size of 7 mm.
[FIG. 27] FIG. 27 shows the results of examination of visualization of uptake of ¹⁰BSH in PC3-Anxal-positive/negative cancer cells by immunofluorescent staining.
[FIG. 28] FIG. 28 shows the results of examination of visualization of uptake of ¹⁰BSH in PC3-Anxal-positive/negative cancer cells by immunofluorescent staining.
[FIG. 29] FIG. 29 shows the results of examination of visualization of uptake of ¹⁰BSH in PC3, SAS, and MG-63 cells by immunofluorescent staining.
[FIG. 30] FIG. 31 shows the results of examination of visualization of uptake of ¹⁰BSH in PC3, SAS, and MG-63 cells by immunofluorescent staining.
[FIG. 31] FIG. 31 shows the results of examination of visualization of uptake of ¹⁰BSH in PC3, SAS, and MG-63 cells by immunofluorescent staining.
[FIG. 32] FIG. 32 shows the results of fluorescence accumulation at the time of sacrifice of prostate cancer-bearing mice to which various ¹⁰B drugs have been administered.
[FIG. 33] FIG. 33 shows the results of visualization of intratissue ¹⁰BSH uptake by immunohistochemical staining with an anti-¹⁰BSH antibody in each tissue 24 hours after administration of various ¹⁰B drugs.
[FIG. 34] FIG. 34 shows the results of visualization of intratissue ¹⁰BSH uptake by immunohistochemical staining with an anti-¹⁰BSH antibody in each tissue 24 hours after administration of various ¹⁰B drugs.
[FIG. 35] FIG. 35 shows the results of visualization of intratissue ¹⁰BSH uptake by immunohistochemical staining with an anti-¹⁰BSH antibody in each tissue 24 hours after administration of various ¹⁰B drugs.
[FIG. 36] FIG. 36 shows the results of change over time in accumulation of ¹⁰BSH in prostate cancer-bearing mice to which a ¹⁰BSH-vc-dhp7 drug has been administered.
[FIG. 37] FIG. 37 shows the results of accumulation of ¹⁰BSH in each organ in prostate cancer-bearing mice 24 hours after administration of a ¹⁰BSH-vc-dhp7 drug.
[FIG. 38] FIG. 38 shows the results of comparison between the antitumor effect in the EMCS-¹⁰BSH-vc-dhp7 administration and BNCT treatment group and the antitumor effect in the ¹⁰BSH and BNCT treatment group in an ANXA1-negative prostate cancer (PC3-ANXA1-) model and an ANXA 1-positive head and neck cancer (SAS) model.
[FIG. 39] FIG. 39 shows the results of comparison between the antitumor effect in the EMCS-¹⁰BSH-vc-dhp7 administration and BNCT treatment group and the antitumor effect in the ¹⁰BSH and BNCT treatment group in an ANXA1-negative prostate cancer (PC3-ANXA1-) model and an ANXA 1-positive head and neck cancer (SAS) model.
[FIG. 40] FIG. 40 shows cytotoxicity to Anxa1 positive/negative PC3 cells and IC50 values.
[FIG. 41] FIG. 41 shows the results of reduction of luciferase-luminescent tumors and change in body weight in treatment with vcMMAE or vcMMAE-dhp7.
[FIG. 42] FIG. 42 shows the appearance and HE staining of resected tumors and the post-treatment blood test results in treatment with vcMMAE alone or vcMMAE-dhp7.
[FIG. 43] FIG. 43 shows the appearance and HE staining of resected tumors and the post-treatment blood test results in treatment with vcMMAE alone or vcMMAE-dhp7.
[FIG. 44] FIG. 44 shows the results of immunostaining of resected tumors in treatment with vcMMAE alone or vcMMAE-dhp7.
[FIG. 45] FIG. 45 shows the results of immunostaining of resected tumors in treatment with vcMMAE alone or vcMMAE-dhp7.
[FIG. 46] FIG. 46 shows the results of reduction of luciferase-luminescent tumors and change in body weight in treatment with vcMMAE alone or vcMMAE-dhp7.
[FIG. 47] FIG. 47 shows the appearance of resected tumors in treatment with vcMMAE alone or vcMMAE-dhp7.
[FIG. 48] FIG. 48 shows the appearance of resected tumors in treatment with vcMMAE alone or vcMMAE-dhp7.

### DESCRIPTION OF EMBODIMENTS

### 1. Peptide of Present Invention

The present invention provides a peptide that includes specific amino acids and binds to annexin A1. More specifically, the present invention provides a peptide comprising an amino acid sequence represented by any one of the formulas (I) to (V) (wherein each amino acid symbol immediately following the symbol [D] represents the D-form of the corresponding amino acid):
(I) [D](X1)[D](X2)[D](X3)[D]E[D]V[D]R[D]S, wherein X1 represents H or Q, X2 represents P or S, and X3 represents N or K (SEQ ID NO: 1),
(II) [D]Q[D](X2)[D]A[D]T[D](X5)[D]L[D]K, wherein X2 represents Y or L, and X5 represents N, K, or Y (SEQ ID NO: 2),
(III) [D](X1)[D]T[D]S[D](X4)[D](X5)[D]T[D]L, wherein X1 represents S or R, X4 represents R or W, and X5 represents N or I (SEQ ID NO: 3),
(IV) any one of the amino acid sequences (I) to (III), which has one or several amino acid insertions, one or several amino acid substitutions, or one or several amino acid deletions, or a combination thereof, and
(V) an amino acid sequence that is a retro-inverso of any one of the amino acid sequences (I) to (IV).

One-letter and three-letter codes for amino acids as defined according to the IUPAC-IUB Joint Commission on Biochemical Nomenclature (JCBN) are used herein.

As used herein, amino acid sequences of (linear) peptides are described with N-terminal amino acids on the left and C-terminal amino acids on the right, in accordance with the conventional peptide description. Each amino acid symbol immediately following the symbol [D] in an amino acid sequence represents the D-form of the corresponding amino acid, and each amino acid symbol immediately not followed by the symbol [D] in an amino acid sequence represents the L-form of the corresponding amino acid, unless the context indicates to the contrary. Peptides including any one of the amino acid sequences (I) to (V) are herein collectively referred to as "the peptide of the present invention." The peptide of the present invention may consist of any one of the amino acid sequences (I) to (V).

In one embodiment, the amino acid sequence (I) is (I')
[D](X1)[D](X2)[D]N[D]E[D]V[D]R[D]S, wherein X1 represents H or Q, and X2 represents P or S (SEQ ID NO: 4).

In one embodiment, the amino acid sequence (I) is [D]H[D]P[D]N[D]E[D]V[D]R[D]S (SEQ ID NO: 5), [D]Q[D]P[D]N[D]E[D]V[D]R[D]S (SEQ ID NO: 6), [D]H[D]S[D]N[D]E[D]V[D]R[D]S (SEQ ID NO: 7), [D]Q[D]S[D]N[D]E[D]V[D]R[D]S (SEQ ID NO: 8), or [D]Q[D]P[D]K[D]E[D]V[D]R[D]S (SEQ ID NO: 9), preferably [D]H[D]P[D]N[D]E[D]V[D]R[D]S (SEQ ID NO: 10).

In one embodiment, the amino acid sequence (II) is [D]Q[D]Y[D]A[D]T[D]N[D]L[D]K (SEQ ID NO: 11), [D]Q[D]Y[D]A[D]T[D]K[D]L[D]K (SEQ ID NO: 12), or [D]Q[D]L[D]A[D]T[D]Y[D]L[D]K (SEQ ID NO: 13), preferably [D]Q[D]Y[D]A[D]T[D]N[D]L[D]K (SEQ ID NO: 14).

In one embodiment, the amino acid sequence (III) is [D]S[D]T[D]S[D]R[D]N[D]T[D]L (SEQ ID NO: 15) or [D]R[D]T[D]S[D]W[D]I[D]T[D]L (SEQ ID NO: 16), preferably [D]S[D]T[D]S[D]R[D]N[D]T[D]L (SEQ ID NO: 17).

The amino acid sequence (IV) has one to several (such as 2, 3, 4, 5, 6, 7, 8, or 9), more specifically, 1 to 5, 1 to 4, 1 to 3, 1 or 2, or 1 of amino acid insertions (or additions), amino acid substitutions, or amino acid deletions, or combinations thereof. An amino acid to be inserted or substituted, for example, may be either a D-amino acid or an L-amino acid, but is preferably a D-amino acid in terms of protease resistance and the like. The peptide of the present invention including the amino acid sequence (IV) may include a partial sequence consisting of four consecutive amino acids, a partial sequence consisting of five consecutive amino acids, or a partial sequence consisting of six consecutive amino acids in the amino acid sequences (I) to (III).

An amino acid to be inserted (or added), substituted, or deleted in the amino acid sequence (IV) may be any amino acid as described later. The amino acid may be an L-amino acid or a D-amino acid, and may be, for example, a naturally occurring L-amino acid as described later, or a D-amino acid which is an optical isomer thereof. The amino acid may also be subjected to various chemical modifications as described later.

An amino acid may be inserted into (or added to) the amino acid sequence (IV) either at the N-terminus or C-terminus of the original sequence or within the sequence, as long as the resulting sequence can bind to ANXA1.

An amino acid may be substituted in the amino acid sequence (IV) preferably with an amino acid having similar physicochemical properties ("a similar amino acid"), for example, an amino acid categorized in the same group such as an aromatic amino acid (Phe (F), Trp (W), or Tyr (Y)), an aliphatic amino acid (Ala (A), Leu (L), Ile (I), or Val (V)), a polar amino acid (Gln (Q) or Asn (N)), a basic amino acid (Lys (K), Arg (R), or His (H)), an acidic amino acid (Glu (E) or Asp (D)), an amino acid having a hydroxyl group (Ser (S) or Thr (T)), or an amino acid having a small side chain (Gly (G), Ala (A), Ser (S), Thr (T), or Met (M)). Substitution with such a similar amino acid is expected not to change the phenotype of the protein (that is, to be conservative amino acid substitution). Specific examples of the conservative amino acid substitution are well-known in the art and described in various documents (see Bowie et al., Science, 247:1306-1310 (1990), for example). As used herein, the conservative amino acid substitution may include substitution of a D-amino acid with an L-amino acid which is an optical isomer thereof. The conservative amino acid substitution may be substitution between L-amino acids, substitution between D-amino acids, or substitution between an L-amino acid and a D-amino acid.

An amino acid may be deleted from the amino acid sequence (IV) at any position of the original sequence, either at the N-terminus or C-terminus of the original sequence or within the sequence, as long as the resulting sequence can bind to ANXA1.

The length of the peptide of the present invention is not particularly limited as long as it can bind to ANXA1. For example, the peptide may include at least two, at least three, at least four, at least five, at least six, at least seven, at least eight, at least nine, at least ten, at least 11, at least 12, at least 13, at least 14, or at least 15 amino acids. The peptide of the present invention may consist of, for example, up to 50, up to 45, up to 40, up to 35, up to 30, up to 25, up to 20, up to 19, up to 18, up to 17, up to 16, up to 15, up to 14, up to 13, up to 12, up to 11, up to 10, up to nine, up to eight, or up to seven amino acids. Alternatively, the peptide of the present invention may have, for example, a length of 3 to 10, 3 to 15, 3 to 20, 3 to 25, 3 to 30, 3 to 40, 3 to 50, 4 to 10, 4 to 15, 4 to 20, 4 to 25, 4 to 30, 4 to 40, 4 to 50, 5 to 10, 5 to 15, 5 to 20, 5 to 25, 5 to 30, 5 to 40, 5 to 50, 6 to 10, 6 to 15, 6 to 20, 6 to 25, 6 to 30, 6 to 40, 6 to 50, 7 to 10, 7 to 15, 7 to 20, 7 to 25, 7 to 30, 7 to 40, 7 to 50, 8 to 10, 8 to 15, 8 to 20, 8 to 25, 8 to 30, 8 to 40, or 8 to 50 amino acids. The peptide of the present invention may have, for example, a length of three, four, five, six, seven, eight, nine, ten, 11, 12, 13, 14, or 15 amino acids.

The peptide of the present invention may be constituted by a combination of D- and L-amino acids. More particularly, the amino acids that constitute the peptide including any one of the amino acid sequences (I) to (V) may all be D-amino acids, or may include L-amino acids in addition to D-amino acids. The L-amino acids may be naturally occurring L-amino acids, and examples thereof include glycine, alanine, leucine, proline, phenylalanine, tyrosine, methionine, serine, threonine, cysteine, aspartic acid, glutamic acid, asparagine, glutamine, lysine, arginine, hydroxylysine, histidine, tryptophan, and valine, which are all L-forms. Examples of the D-amino acids include optical isomers of the L-amino acids as described above. Glycine, which is an optically inactive amino acid, can be understood herein as a D/L-amino acid, unless the context indicates to the contrary.

The peptide of the present invention may include modified or unusual amino acids such as those mentioned in 37 C.F.R. 1.821-1.822. The modified or unusual amino acids are not particularly limited, and examples thereof include 2-aminoadipic acid, 3-aminoadipic acid, β-alanine, 2-aminobutyric acid, 4-aminobutyric acid, 6-aminocaproic acid, 2-aminoheptanoic acid, 2-aminoisobutyric acid, 3-aminoisobutyric acid, 2-aminopimelic acid, 2,4-diaminobutyric acid, desmosine, 2,2'-diaminopimelic acid, 2,3-diaminopropionic acid, N-ethylglycine, N-ethylasparagine, hydroxylysine, allo-hydroxylysine, 3-hydroxyproline, 4-hydroxyproline, isodesmosine, allo-isoleucine, N-methylglycine, N-methylisoleucine, 6-N-methyllysine, N-methylvaline, norvaline, norleucine, and ornithine.

The amino terminus and/or the carboxy terminus of the peptide of the present invention may be modified. Modification of the amino terminus may be, for example, methylation (e.g., - NHCH₃ or -N(CH₃)₂), acetylation (e.g., by acetic acid or its halogenated derivative (such as α-chloroacetic acid, α-bromoacetic acid, or α-iodoacetic acid)), urea formation, carbamate formation, formylation, tert-butyloxycarbonylation, or 9-fluorenylmethyloxycarbonylation. Alternatively, any protecting group such as a benzyloxycarbonyl group, a carboxylate functional group (RCOO-), or a sulfonyl functional group (R-SO₂-), wherein R is selected from alkyl, aryl, heteroaryl, alkylaryl, and the like, may be introduced at the amino terminus.

Modification of the carboxy terminus includes amidation (-CONH₂) and esterification (-COOR). Here, C₁₋₆ alkyl groups such as methyl, ethyl, n-propyl, isopropyl, and n-butyl; C₃₋₈ cycloalkyl groups such as cyclopentyl and cyclohexyl; C₆₋₁₂ aryl groups such as phenyl and α-naphthyl; phenyl-C₁₋₂ alkyl groups such as benzyl and phenethyl; C₇₋₁₄ aralkyl groups such as an α-naphthyl-C₁₋₂ alkyl group; and a pivaloyloxymethyl group are used as R in the ester.

The peptide of the present invention may also be variously modified at a position other than the N- or C-terminus. Chemical modification may be, for example, methylation, acetylation, or phosphorylation. When the peptide of the present invention has a carboxyl group (carboxylate) at a position other than the C-terminus, the carboxyl group may be amidated or esterified. For example, the C-terminal ester as described above is used as the ester in this case. Alternatively, a substituent on the side chain of an amino acid in the molecule (such as -OH, - SH, an amino group, an imidazole group, an indole group, or a guanidino group) may be protected by a suitable protecting group (e.g., a C₁₋₆ alkanoyl group (a C₁₋₆ acyl group) such as a formyl group or an acetyl group).

For the amino acid sequence (V), the retro-inverso isomer of the peptide refers to a peptide in which the chirality of each amino acid residue is inverted ("inverso") and the direction of the amino acid sequence is reversed ("retro") with respect to the original peptide. The retro-inverso isomer is known to have a structure and a function similar to those of the original peptide (e.g., Acc. Chem. Res., 1993, 26(5), pp. 266-273; PLoS One. 2013 Dec 2;8(12): e80390). Specific examples of the amino acid sequence (V) include a sequence that is a retro-inverso of the sequence (I), such as SRVENPH (SEQ ID NO: 18), SRVENPQ (SEQ ID NO: 19), SRVENSH (SEQ ID NO: 20), SRVENSQ (SEQ ID NO: 21), or SRVEKPQ (SEQ ID NO: 22); a sequence that is a retro-inverso of the sequence (II), such as KLNTAYQ (SEQ ID NO: 23), KLKTAYQ (SEQ ID NO: 24), or KLYTALQ (SEQ ID NO: 25); and a sequence that is a retro-inverso of the sequence (III), such as LTNRSTS (SEQ ID NO: 26) or LTIWSTR (SEQ ID NO: 27).

The peptide of the present invention may include two or more sequences selected from the amino acid sequences (I) to (V). In one embodiment, the peptide of the present invention includes a tandem repeat of any one of the amino acid sequences (I) to (V) (that is, a structure in which identical sequence moieties are directly linked to each other). In another embodiment, the peptide of the present invention includes a structure in which the two or more different amino acid sequences (I) to (V) are directly linked to each other. Alternatively, the peptide of the present invention may constitute a multivalent peptide by a dendrimer.

The peptide of the present invention may be a free peptide or a salt form. Examples of the salt of the peptide of the present invention include pharmaceutically acceptable acid or base addition salts. Acid addition salts include salts with inorganic acids such as hydrochloric acid, sulfuric acid, nitric acid, and phosphoric acid; and salts with organic acids such as acetic acid, malic acid, succinic acid, tartaric acid, and citric acid. Base addition salts include salts with alkali metals such as sodium and potassium; salts with alkaline earth metals such as calcium and magnesium; ammonium salts; and salts with amines such as triethylamine.

The peptide of the present invention can bind to annexin A1. ANXA1, also known as lipocortin 1, is a known protein belonging to the annexin family. DNA nucleotide sequences and amino acid sequences of ANXA1 are known for various biological species. The peptide of the present invention can bind to the N-terminal region of ANXA1. More particularly, the peptide of the present invention can bind to the 15 N-terminal residues (MAMVSEFLKQAWFIE (SEQ ID NO: 28)) of ANXA1 (such as human ANXA1 or mouse ANXA1). In one embodiment, the peptide of the present invention can bind to the 15 N-terminal residues (MAMVSEFLKQAWFIE (SEQ ID NO: 28)) of ANXA1 (such as human ANXA1 or mouse ANXA1) within five minutes at 37°C. The peptide of the present invention can have an equilibrium dissociation constant (KD value) of preferably 10⁻⁶ M or less, more preferably 5.0 × 10⁻⁷ M or less, when, for example, its intermolecular interaction with mouse or human ANXA1 is measured by biolayer interferometry (BLI).

The peptide of the present invention can be produced according to a known peptide synthesis. The peptide synthesis may be, for example, either solid-phase synthesis or liquid-phase synthesis. The intended peptide may also be produced by condensing a partial peptide or amino acid moiety that can constitute the peptide of the present invention with the remaining moiety, and eliminating a protecting group when the product has the protecting group.

Here, condensation and elimination of the protecting group can be performed by a method known per se, such as a method as described in (1) to (8) below:
(1) M. Bodanszky & M. A. Ondetti, Peptide Synthesis, Interscience Publishers, New York (1966),
(2) Schroeder & Luebke, The Peptide, Academic Press, New York (1965),
(3) Nobuo Izumiya et al., Peptide Gosei No Kiso To Jikken [Fundamentals and Experiments of Peptide Synthesis], Maruzen Co., Ltd. (1975),
(4) Haruaki Yajima and Shunpei Sakakibara, Seikagaku Jikken Koza [Course of Biochemical Experiments] 1, Tanpakushitsu No Kagaku [Protein Chemistry] IV 205 (1977),
(5) Haruaki Yajima (supervisor), Zoku Iyakuhin No Kaihatsu [Development of Pharmaceuticals, Second Series], Vol. 14, Peptide Gosei [Peptide Synthesis], Hirokawa Shoten,
(6) Stewart, J. M. & Young, J. D., "Solid phase peptide synthesis (2nd ed.)", Pierce Chemical Company, Rockford (1984),
(7) Atherton, E. & Sheppard, R. C., "Solid Phase peptide synthesis: a practical approach", IRL Press, Oxford (1989), and
(8) "Fmoc Solid Phase Peptide Synthesis: A Practical Approach (Practical Approach Series)", Oxford University Press (2000).

The peptide obtained in this manner can be purified and isolated by a known purification method. Here, examples of the purification method include solvent extraction, distillation, column chromatography, liquid chromatography, recrystallization, and a combination thereof. When the peptide obtained by the above method is a free peptide, the free peptide can be converted to a suitable salt by a known method or its equivalent. Conversely, when the peptide is obtained as a salt, the salt can be converted to a free peptide or another salt by a known method or its equivalent.

The peptide of the present invention can be orally or parenterally administered to a subject as described below safely. Parenteral administration includes intravenous administration, intramuscular administration, subcutaneous administration, intraorgan administration, intranasal administration, intradermal administration, ophthalmic administration, intracerebral administration, rectal administration, vaginal administration, intraperitoneal administration, intratumoral administration, administration to a site proximal to the tumor, and direct administration to the lesion.

As described above, annexin A1 is known to have extremely high specificity among currently known tumor vessel-specific marker molecules. It is intracellularly expressed in normal cells, but is strongly expressed on the luminal surface in contact with the blood flow in tumor neovascular endothelial cells (Oh et al., Nature 429: 629-35, 2004). Accordingly, the peptide of the present invention can selectively bind to angiogenic tumors in vivo. Further, ANXA1 is expressed on the blood side of neovascular endothelial cells formed in the tumor. When ANXA1 binds to a ligand such as the peptide of the present invention on the blood side, it transports the ligand to the basal side by transcytosis and actively releases the ligand to the interstitial tissue in which cancer cells are present. Accordingly, the peptide of the present invention can target malignant tumors in vivo. In this context, the peptide of the present invention is useful for, for example, applications such as targeting, (molecular) imaging, and companion diagnosis of malignant tumors.

For example, in the companion diagnosis, an annexin A1-positive cancer tissue (a cancer tissue containing annexin A1-positive cells, or an annexin A1-positive (solid) cancer) can be identified by, for example, selecting a specific cell group in a cancer tissue and determining how much or whether the cancer tissue contains annexin A1-positive cells. The selection and determination can be performed according to a known method. Specific examples thereof include immunohistochemical staining. This method is performed by staining annexin A1-positive cells in a cancer tissue with an anti-human annexin A1 antibody (fluorescent or colorimetric staining), and determining whether the cells are positive or negative, based on the amount of the dye emitted from the stained cells (e.g., the amount of fluorescence or visible light). Typically, the staining can be performed utilizing an annexin A1-binding molecule. Thus, in one embodiment, the present invention relates to a companion diagnostic agent for the neutron capture therapy agent or cancer chemotherapeutic agent of the present invention, the companion diagnostic agent including an annexin A1-binding molecule (hereinafter also referred to as "the diagnostic agent of the present invention"). Efficacy of the cancer therapeutic or prophylactic agent of the present invention can be predicted by a method including the step of bringing an annexin A1-binding molecule into contact with cells in a cancer tissue of a subject.

The annexin A1-binding molecule is not particularly limited as long as it is a molecule capable of binding to (preferably, specifically binding to) annexin A1. Examples of the annexin A1-binding molecule include antibodies, more specifically, immunoglobulins, Fab, F(ab')2, minibodies, scFv-Fc, Fv, scFv, diabodies, triabodies, tetrabodies, and monobodies.

A series of operations for the selection and determination can be performed by microscopy, for example. Determination on whether the cells are positive or negative is performed based on the amount of the dye emitted from the stained cells according to a known method. The determination can be performed according to a known technique. For example, the amount of the dye emitted from negative control cells (specifically, for example, cells stained with an isotype control antibody) is defined as background. Based on an amount of the dye sufficiently higher than the background as reference, cells emitting a smaller amount of the dye than the reference are determined as negative cells, and cells emitting a higher amount of the fluorescent dye than the reference are determined as positive cells. As a more convenient method, it is also possible to use a method involving reverse transcription quantitative PCR of annexin A1 for mRNA extracted from a cancer tissue and determination on whether the cells are positive or negative based on the relative amount of annexin A1 mRNA.

In one embodiment, a subject having a cancer tissue containing annexin A1-positive cells (or a subject suffering from a cancer containing annexin A1-positive cells (an annexin A1-positive cancer)) is selected from subjects having a cancer tissue (subjects suffering from cancer), and the cancer therapeutic or prophylactic agent of the present invention is administered to the selected subject. The cancer therapeutic or prophylactic agent of the present invention can be used for administration to a subject having a cancer tissue containing annexin A1-positive cells (or a subject suffering from a cancer containing annexin A1-positive cells (an annexin A1-positive cancer)) selected from subjects having a cancer tissue (subjects suffering from cancer).

### 2. Conjugate of Present Invention

The present invention also provides a conjugate including the peptide of the present invention as described above and one or more components (hereinafter also referred to as "the conjugate of the present invention"). The components are not particularly limited as long as they can be linked to the peptide of the present invention. The components can be suitable for administration to animals (such as humans) and can also have any function in the bodies of the animals. The components may be naturally occurring substances or non-naturally occurring substances.

Examples of the components include, but are not limited to, biological materials (such as cells, phages, and viruses), oligonucleotides and nucleic acids (such as DNAs, RNAs, and DNA/RNA chimeras), peptides, polypeptides, proteins, antibodies, lipids, polysaccharides, small molecules (organic or inorganic) (such as boron and gadolinium compounds), particles (such as gold particles and various nanoparticles), and combinations thereof.

The components can have a given functionality at the target site in the body of an animal (such as a human). The type of the functionality is not particularly limited. The conjugate of the present invention can target malignant tumors, for example, by the action of the moiety corresponding to the peptide of the present invention. Thus, preferred examples of the functionality include having antitumor activity and detectability. Accordingly, the components may be, for example, a small molecule, an anticancer agent, and a detectable substance.

### (Boron Compound)

The boron compound is not particularly limited as long as it is a compound containing a boron atom. Examples thereof include compounds containing boron-10 nuclide (¹⁰B). The boron compound is preferably one that can be used for neutron capture therapy. Specific examples thereof include R-B(OH)₂ (a boronic acid, wherein R is a substituent selected from, for example, alkyl, aryl, heteroaryl, and alkylaryl), BPA (p-boronophenylalanine), borax, PCPB (4-carboxybenzeneboronic acid), BODIPY (boron-dipyrromethene), and a boron cluster.

As used herein, the boron cluster mainly refers to an ionic or non-ionic substance having a structure obtained by assembly and bonding of multiple boron atoms into one cluster, and is preferably a boron cluster having 3 to 20 boron atoms, more preferably a boron cluster having 8 to 20 boron atoms, particularly preferably a boron cluster having 10 to 12 boron atoms.

Examples of the boron cluster include, but are not limited to, boron hydride compounds such as decaborane (B₁₀H₁₄), decahydrodecaborate ([B₁₀H₁₀]²⁻) (GB-10) (also called sodium decahydrodecaborate), dodecaborate ([B₁₂H₁₂]²⁻), and nido,nido-Octadecaborane(22) (B₁₈H₂₂, CAS RN: 21107-56-2).

The boron cluster may also contain atoms other than boron such as carbon, nitrogen, oxygen, and sulfur atoms as its constituent backbone atoms. The boron cluster contains preferably 0 to 5, more preferably 0 to 2, of other atoms such as carbon, nitrogen, oxygen, and sulfur atoms.

Examples of the boron cluster containing atoms other than boron in the basic backbone include carboranes containing boron and carbon atoms (including so-called carborane isomers). Examples of the carboranes include, but are not limited to, known carboranes such as closo-carborane containing one carbon atom ([CB₁₁H₁₂]⁻), closo-carborane (C₂B₁₀H₁₂) or nido-carborane ([C₂B₉H₁₁]⁻) containing two carbon atoms, or sandwich-type carborane represented by the molecular formula M(C₂B₉H₁₀)₂, for example. In the above molecular formulas, M represents a transition metal element and can be, for example, Fe, Ni, Co, or Mo. That is, the boron cluster may be a metal complex with a transition metal such as Fe, Ni, Co, or Mo.

The boron cluster is preferably ionic, and is also preferably water-soluble. Examples of the ionic or water-soluble boron cluster include dodecaborate ([B₁₂Hi₂]²⁻), dodecaborate having a thiol group ([B₁₂H₁₁SH]²⁻ or BSH (sometimes also referred to as (sodium) borocaptate or mercaptoundecahydrododecaborate)), dodecaborate having a hydroxyl group ([B₁₂H₁₁OH]²⁻), and dodecaborate having an amino group ([B₁₂H₁₁NH₃]⁻).

### (Gadolinium Compound)

The gadolinium compound is not particularly limited as long as it is a compound containing a gadolinium atom. Examples thereof include ¹⁵⁷Gd and compounds containing ¹⁵⁷Gd.

### (Anticancer Agent)

As used herein, the anticancer agent refers to a drug intended to suppress malignant tumor (cancer) growth. The mechanism of action of the anticancer agent is not particularly limited. The anticancer agent may be an antimetabolite, an alkylating agent, an anticancer antibiotic, a microtubule inhibitor, a platinum product, a topoisomerase inhibitor, a molecular target drug, or the like. The conjugate of the present invention may include two or more identical or different anticancer agents.

The antimetabolite may be, for example, an antifolate, a dihydropteroate synthase inhibitor, a dihydrofolate reductase inhibitor (a DHFR inhibitor), a pyrimidine metabolic inhibitor, a thymidylate synthase inhibitor, a purine metabolic inhibitor, an IMPDH inhibitor, a ribonucleotide reductase inhibitor, a ribonucleotide reductase inhibitor, a nucleotidic analog, or L-asparaginase. Specific examples of the antimetabolite include enocitabine (Sunrabin), capecitabine (Xeloda), carmofur (Mifurol), cladribine (Leustatin), gemcitabine (Gemzar), cytarabine (Cylocide), cytarabine ocfosphate (Starasid), tegafur (Atilon, Aftfur, Tefsiel, Ftorafur, or Lunacin, etc.), tegafur-uracil (UFT), tegafur-gimeracil-oteracil potassium (TS-1), doxifluridine (Furtulon), nelarabine (Arranon G), hydroxycarbamide (Hydrea), fluorouracil (5-FU, Carzonal, Bennan, Lunachol, or Lunabon), fludarabine (Fludara), pemetrexed (Alimta), pentostatin (Coforin), mercaptopurine (Leukerin), and methotrexate.

Specific examples of the alkylating agent include nitrogen mustard alkylating agents such as cyclophosphamide (Endoxan), ifosfamide (Ifomide), melphalan (Alkeran), busulfan, and thiotepa (Tespamin); and nitrosourea alkylating agents such as nimustine (Nidran), ranimustine (Cymerin), dacarbazine, procarbazine (procarbazine hydrochloride), temozolomide (Temodal), carmustine (Gliadel), streptozotocin (Zanosar), and bendamustine (Treakisym).

Specific examples of the anticancer antibiotic include actinomycin D (Cosmegen), aclarubicin (Aclacinon), amrubicin (Calsed), idarubicin (Idamycin), epirubicin (epirubicin hydrochloride, Farmorubicin), zinostatin stimalamer (Smancs), daunorubicin (daunomycin), doxorubicin (Adriacin), pirarubicin (Pinorubin, Therarubicin), bleomycin (Bleo), peplomycin (Pepleo), mitomycin C (mitomycin), mitoxantrone (Novantrone), and liposomal doxorubicin (Doxil).

Examples of the microtubule inhibitor include vinca alkaloid microtubule polymerization inhibitors such as vinblastine (Exal), vincristine (Oncovin), and vindesine (Fildesin); and taxane microtubule depolymerization inhibitors such as paclitaxel (Taxol), docetaxel (Taxotere), and vedotin. Further examples thereof include monomethyl auristatin E (MMAE).

Examples of the platinum product include oxaliplatin (Elplat), carboplatin (carboplatin, Carbomerck, or Paraplatin), cisplatin (IA-call, Conabli, or cisplatin, etc.), and nedaplatin (Aqupla).

Examples of the topoisomerase inhibitor include topoisomerase I inhibitors such as camptothecin and its derivatives (such as irinotecan (Campto), nogitecan (Hycamtin), and SN-38); and topoisomerase II inhibitors such as anthracycline drugs such as doxorubicin (adriacin), epipodophyllotoxin drugs such as etoposide (Lastet or VePesid), and quinolone drugs such as levofloxacin (Cravit) or ciprofloxacin (Ciproxan).

Examples of the molecular target drug include regorafenib (Stivarga), cetuximab (Erbitux), panitumumab (Vectibix), ramucirumab (Cyramza), gefitinib (Iressa), erlotinib (Tarceva), afatinib (Giotrif), crizotinib (Xalkori), alectinib (Alecensa), ceritinib, lenvatinib (Lenvima), trastuzumab (Herceptin), lapatinib (Tykerb), pertuzumab (Perjeta), sunitinib (Sutent), sorafenib (Nexavar), axitinib (Inlyta), pazopanib (Votrient), nivolumab (Opdivo), pembrolizumab, ipilimumab (Yervoy), vemurafenib (Zelboraf), everolimus (Afinitor), temsirolimus (Torisel), rituximab (Rituxan), bevacizumab (Avastin), and geldanamycin.

The anticancer agent may also be an anti-angiogenic agent. The anti-angiogenic agent can inhibit a vascular endothelial growth factor (VEGF) or another angiogenic factor, or a receptor thereof. Specific examples of the anti-angiogenic agent include angiostatin, endostatin, metastatin, anti-VEGF antibodies (such as Avastin), and VEGFR-2 inhibitors (such as SU5416 and SU6668).

### (Detectable Substance)

As used herein, the detectable substance refers to any substance that allows detection of the conjugate of the present invention including the detectable substance. Preferably, the detectable substance allows the conjugate of the present invention to be detected in vivo directly or indirectly using a suitable visualization or imaging means. Examples of the visualization or imaging means include, but are not limited to, radiography, computed tomography (CT), magnetic resonance imaging (MRI), ultrasonography, scintigraphy, positron emission tomography (PET), radionuclide therapy, endoscopy, and laparoscopy. The detectable substance may be, for example, a radioisotope, an MRI contrast enhancer (such as a paramagnetic ion), a radiopaque substance, a contrast medium, or a fluorescent substance.

Examples of the radionuclide useful for PET include ¹⁸F, ⁵¹Mn, ^{52m}Mn, ⁵²Fe, ⁵⁵Co, ⁶²Cu, ⁶⁴Cu, ⁶⁸Ga , ⁷²As, ⁷⁵Br, ⁷⁶Br, ^{82m}Rb, ⁸³Sr, ⁸⁶Y, ⁸⁹Zr, ^{94m}Tc, ¹¹⁰In, ¹²⁰I, and ¹²⁴I. Examples of the radionuclide useful for detecting γ-rays include ⁵¹Cr, ⁵⁷Co, ⁵⁸Co, ⁵⁹Fe , ⁶⁷Cu, ⁶⁷Ga, ⁷⁵Se, ⁹⁰Y, ⁹⁷Ru, ^{99m}Tc, ¹¹¹In, ^{114m}In, ¹²³I, ¹²⁵I, ¹³¹I, ¹⁶⁹Yb, ¹⁷⁷Lu, ¹⁹²Ir, ¹⁹⁷Hg, ¹⁹⁸AU, ²⁰¹Tl, ²¹¹At, ²²⁵Ac, and ²²³Ra.

Suitable examples of the paramagnetic ion include chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III), and erbium (III), with gadolinium being particularly preferred. Metals such as lanthanum (III), gold (III), lead (II), and bismuth (III) are also useful for applications such as X-ray imaging.

Examples of the radiopaque substance and the contrast medium include iodine compounds (such as organic iodic acids such as iodocarboxylic acid, iodoform, triiodophenol, and tetraiodoethylene), barium compounds (such as barium sulfate), gallium compounds (such as gallium citrate), and thallium compounds (such as thallium chloride).

Examples of the fluorescent substance include rhodamine, fluorescein, Cy dyes (such as Cy3, Cy5, Cy5.5, Cy7, and Cy7.5), Alexa (registered trademark) Fluor, phycoerythrin (PE), allophycocyanin (APC), and their derivatives. Near-infrared fluorescent reagents such as indocyanine are also preferred examples of the fluorescent substance.

### (Conjugation Between Peptide of Present Invention and Components)

There are no particular limitations to the mode of conjugation between the peptide of the present invention and the one or more components in the conjugate of the present invention. The conjugation may be either direct conjugation or indirect conjugation via a linker or the like. The conjugation may be covalent conjugation, non-covalent conjugation, or a combination thereof. The one or more components may be directly or indirectly conjugated to the N-terminus, the C-terminus, or other positions of the peptide of the present invention. Conjugation between a peptide and other components (or a second peptide) is well-known in the art, and such conjugation may also be performed by any known means in the conjugate of the present invention.

In one example, when the conjugation is performed via a linker, it is possible to use a known crosslinker such as NHS ester, Sulfo-NHS ester, imide ester, maleimide, carbodiimide, allyl azide, diazirine, isocyanide, psoralen, or a combination thereof (such as a homobifunctional crosslinker or a heterobifunctional crosslinker). Examples of the homobifunctional crosslinker include DST, BS2G, DSG, BS3, DSS, DSP, DTSSP, DSSeb, EGS, and Sulfo-EGS. Examples of the heterobifunctional crosslinker include SBA, SIA, Sulfo-SIA, BMPS, SPDP, GMBS, MBS, Sulfo-MBS, ANB-NOS, SMCC, Sulfo-SMCC, PDPH, EMCS (N-(6-maleimidocaproyloxy)succinimide), SMPB, SMPH, LC-SPDP, Sulfo-LC-SPDP, and Sulfo-SNAPAH. It is also possible to use, for example, a peptide linker such as a dipeptide linker (such as a Val-Cit linker). The N-terminus of the linker may be protected with an Fmoc or Alloc group, or the like, or a maleimide group or the like may be introduced into the N-terminus. It is further possible to use two or more of the above-described linkers in combination. The peptide of the present invention may be appropriately modified depending on the crosslinker used. For example, cysteine may be added to the C-terminus of the peptide of the present invention in advance for conjugation with a maleimide linker.

For conjugation between the above-described radioactive substance (nuclide) or paramagnetic ion and the peptide of the present invention, it is possible to use, for example, a suitable chelating agent (such as ethylenediaminetetraacetic acid (EDTA), diethylenetriaminepentaacetic acid (DTPA), or 4,7,10-tetraazacyclododecane-N-N',N",N‴-tetraacetic acid (DOTA)) and/or metallothionein (see e.g., Cumali Aktolun et al., "Nuclear Medicine Therapy: Principles and Clinical Applications", Springer, 2013).

The conjugate of the present invention can be orally or parenterally administered to a subject as described below safely. Parenteral administration includes intravenous administration, intramuscular administration, subcutaneous administration, intraorgan administration, intranasal administration, intradermal administration, ophthalmic administration, intracerebral administration, rectal administration, vaginal administration, intraperitoneal administration, intratumoral administration, administration to a site proximal to the tumor, and direct administration to the lesion.

### 3. Pharmaceutical Composition of Present Invention

The present invention also provides a pharmaceutical composition including the peptide or conjugate of the present invention (hereinafter also referred to as "the pharmaceutical composition of the present invention"). The pharmaceutical composition of the present invention may include a pharmaceutically acceptable carrier. The pharmaceutical composition can be provided as a dosage form suitable for oral or parenteral administration. Parenteral administration includes intravenous administration, intramuscular administration, subcutaneous administration, intraorgan administration, intranasal administration, intradermal administration, ophthalmic administration, intracerebral administration, rectal administration, vaginal administration, intraperitoneal administration, intratumoral administration, administration to a site proximal to the tumor, and direct administration to the lesion. The administration can be performed using, for example, an injection, an endoscope, or a catheter.

For example, injections or suppositories are used as pharmaceutical compositions for parenteral administration. Injections may include dosage forms such as intravenous injections, subcutaneous injections, intraperitoneal injections, intradermal injections, intramuscular injections, and drip injections. Such injections can be prepared according to a known method. Injections can be prepared by, for example, a method involving dissolving, suspending, or emulsifying the peptide or conjugate of the present invention in a sterile aqueous or oily liquid conventionally used for injections. The aqueous liquid for injection use is, for example, an isotonic solution containing physiological saline, glucose, or other adjuvants, and may be used in combination with a suitable solubilizer such as an alcohol (such as ethanol), a polyalcohol (such as propylene glycol or polyethylene glycol), a nonionic surfactant (such as polysorbate 80 or HCO-50 (polyoxyethylene (50 mol) adduct of hydrogenated castor oil)). The oily liquid for injection use is, for example, sesame oil or soybean oil, and may be used in combination with a solubilizer such as benzyl benzoate or benzyl alcohol. The prepared injection is preferably filled in a suitable ampoule. Suppositories used for rectal administration can be prepared by mixing the peptide or conjugate of the present invention with a conventional suppository base.

Pharmaceutical compositions for oral administration include solid or liquid dosage forms, specifically, tablets (including sugar-coated tablets and film-coated tablets), pills, granules, powders, capsules (including soft capsules), syrups, emulsions, and suspensions. Such pharmaceutical compositions are produced by a known method and may contain a carrier, a diluent, an excipient, and the like conventionally used in the pharmaceutical art. The carrier or excipient for use in tablets is for example, lactose, starch, sucrose, or magnesium stearate,.

Although the content of the peptide or conjugate of the present invention in the pharmaceutical composition of the present invention varies depending on the form of the pharmaceutical composition (formulation), the amount of the peptide or conjugate of the present invention is typically about 0.01% to 100% by weight, preferably about 0.1% to 50% by weight based on the entire pharmaceutical composition (the entire formulation).

The pharmaceutical composition of the present invention may contain other active ingredients as long as they do not unfavorably interact with the peptide or conjugate of the present invention to be combined with them.

The parenteral or oral pharmaceutical composition is conveniently prepared into a unit dosage form matching the dose of the active ingredient. Examples of such a unit dosage form include tablets, pills, capsules, injections (ampoules), and suppositories. The content of the peptide or conjugate is not particularly limited as long as the desired efficacy and effect can be achieved, but the content is typically, for example, 0.01 mg to 50 g for a unit dosage form, 0.01 mg to 25 g for an injection, and 0.01 mg to 50 g for another dosage form.

The pharmaceutical composition of the present invention can target tumors, in particular, angiogenic malignant tumors, and preferably can allow the peptide or conjugate to accumulate in the tumors. Accordingly, the pharmaceutical composition of the present invention including the anticancer agent, the boron or gadolinium compound, or the like in the conjugate is useful for the treatment or prevention of targeted malignant tumors. The composition of the present invention including the detectable substance or the like in the conjugate is useful for tests or diagnosis of malignant tumors.

Malignant tumors (cancers) may be any types of cancer and may be solid or liquid cancers. Solid cancers may be preferably ANXA1-positive solid cancers, more preferably solid cancers expressing ANXA1 on the cell surface, i.e., angiogenic solid tumors. Examples of solid cancers include brain and central nervous system cancers (such as brain tumor and spinal cord tumor), head and neck cancers (such as laryngeal cancer, oral cancer, salivary gland cancer, paranasal sinus cancer, and thyroid cancer), gastrointestinal cancers (such as gastric cancer, esophageal cancer, small intestine cancer, colon cancer, rectal cancer, anal cancer, hepatic cancer, biliary cancer, and pancreatic cancer), urogenital cancers (such as renal cancer, renal cell carcinoma, bladder cancer, prostate cancer, cancer of the renal pelvis and ureter, gallbladder cancer, bile duct cancer, testicular cancer, penile cancer, uterine cancer, endometrial cancer, uterine sarcoma, cervical cancer, vaginal cancer, vulvar cancer, ovarian cancer, and fallopian tube cancer), respiratory system cancers (such as lung cancer (including small cell lung cancer, non-small-cell lung cancer, and metastatic lung cancer) and bronchial carcinoma), breast cancer, skin cancers (such as malignant melanoma), bone cancers (such as osteosarcoma), and muscle cancers (such as rhabdomyosarcoma). Preferred solid cancers are, for example, skin cancers (such as malignant melanoma), brain and central nervous system cancers (such as treatment-resistant brain tumor and spinal cord tumor), laryngeal cancer, oral cancer, salivary gland cancer, paranasal sinus cancer, thyroid cancer, bladder cancer, prostate cancer, cancer of the renal pelvis and ureter, and osteosarcoma.

Liquid cancers include leukemias, malignant lymphomas, multiple myeloma, and myelodysplastic syndrome. Leukemias include acute myelogenous leukemia, acute lymphocytic leukemia, chronic myelogenous leukemia, and chronic lymphocytic leukemia. Malignant lymphomas are classified into Hodgkin's lymphomas and non-Hodgkin's lymphomas. Non-Hodgkin's lymphomas include B-cell lymphoma, adult T-cell lymphoma, lymphoblastic lymphoma, diffuse large-cell lymphoma, Burkitt's lymphoma, follicular lymphoma, MALT lymphoma, peripheral T-cell lymphoma, and mantle-cell lymphoma.

For example, brain tumors may be preferred targets of the peptide or conjugate of the present invention, because it can overcome the blood-brain tumor barrier efficiently. Brain tumors may be primary brain tumors or metastatic brain tumors. Brain tumors may be benign brain tumors (such as meningioma, pituitary adenoma, and schwannoma) or malignant brain tumors, and are preferably malignant brain tumors. Malignant brain tumors include Grade 2 brain tumors such as astrocytoma and oligodendroglioma; Grade 3 brain tumors such as anaplastic astrocytoma, anaplastic oligodendroglioma, and anaplastic oligoastrocytoma; and Grade 4 brain tumors such as glioblastoma.

The pharmaceutical composition of the present invention can be administered to a subject expressing annexin A1, such as an animal, in particular, a mammal. Examples of the mammal may include, but are not limited to, laboratory animals such as rodents such as mice, rats, hamsters, and guinea pigs, and rabbits; domestic animals such as pigs, cows, goats, horses, sheep, and minks; pets such as dogs and cats; and primates such as humans, monkeys, rhesus monkeys, marmosets, orangutans, and chimpanzees.

The dose of the pharmaceutical composition of the present invention varies depending on factors such as the purpose of administration, the administration target, the disease of interest, the symptom, and the administration route. For example, when the pharmaceutical composition is used for the treatment or prevention of the cancer as described above, typically, a single dose of about 0.01 mg to 50 g/kg body weight of the conjugate of the present invention including the anticancer agent or the boron or gadolinium compound is intravenously injected or orally administered once a week. Alternatively, when the pharmaceutical composition is used for tests or diagnosis of the cancer as described above, typically, about 0.01 mg to 50 g/kg body weight of the conjugate of the present invention including the detectable substance is intravenously injected or orally administered prior to the tests.

### 4. Agents and Reagent of Present Invention

### (Neutron Capture Therapy Agent)

The present invention also provides a neutron capture therapy agent including the conjugate of the present invention (hereinafter also referred to as "the neutron capture therapy agent of the present invention"). The conjugate includes the boron or gadolinium compound as described above. The neutron capture therapy agent of the present invention can accumulate in a malignant tumor (cancer) expressing the above-described ANXA1 on the cell surface by the action of the peptide of the present invention included in the agent. The accumulation refers to selective orientation (binding) to or localization in a cancerous tissue rather than a non-cancerous tissue (such as a normal tissue) in the subject as described above (such as an animal, more specifically, a mammal (such as a human)). Accordingly, the neutron capture therapy agent of the present invention is suitably used for neutron capture therapy (NCT), more particularly, boron neutron capture therapy (BNCT) or gadolinium neutron capture therapy (GdNCT). Thus, the present invention also provides neutron capture therapy including administering to a subject an effective dose of the conjugate of the present invention including the boron or gadolinium compound as described above or the like. The neutron capture therapy of the present invention may be performed for the treatment of a disease from which a subject suffers, or may be performed for the prevention of the disease (such as the prevention of recurrence of the disease).

Although the content of the conjugate of the present invention in the neutron capture therapy agent of the present invention varies depending on the form of the formulation, the amount of the conjugate of the present invention is typically about 0.01% to 100% by weight, preferably about 0.1% to 50% by weight based on the entire formulation.

In the neutron capture therapy, after the neutron capture therapy agent of the present invention has reached within the tumor, the site is irradiated with an effective dose of a low-energy neutron beam such as a thermal or epithermal neutron beam. The site may be irradiated through the skin, or the site may be completely or partially exposed prior to irradiation and then irradiated. The site may also be irradiated in multiple directions at the same time. Administration of the neutron capture therapy agent of the present invention and subsequent irradiation with a thermal or epithermal neutron beam may be repeated as necessary. For example, the irradiation may be performed multiple times at intervals of about several months. Preferably, the irradiation is performed a total of 1 to 10 times.

The neutron capture therapy agent of the present invention can be administered to a mammal suffering from a malignant tumor (cancer) typically within 72 hours before (preferably five minutes to 48 hours before, more preferably 30 minutes to 48 hours before) irradiation with a thermal or epithermal neutron beam. The neutron capture therapy agent may also be administered during irradiation with a thermal or epithermal neutron beam, as necessary. A typical dose of the neutron capture therapy agent of the present invention is in the range of 0.01 mg to 50 g/kg body weight for one irradiation with a thermal or epithermal neutron beam. The time for one irradiation with a thermal or epithermal neutron beam is preferably one minute to five hours, more preferably 10 minutes to two hours. The radiation dose of the thermal or epithermal neutron beam is not particularly limited, and may be a conventional radiation dose used in neutron capture therapy.

Capture therapy using the neutron capture therapy agent of the present invention may be applied as a single treatment, or may be applied together with a conventional surgery or chemotherapy. If desired, after removing tumors to a surgically possible extent, the residual tumors can be destroyed by capture therapy using the neutron capture therapy agent of the present invention.

In neutron capture therapy, the neutron capture therapy agent of the present invention may be used together with, for example, a concomitant drug such as an anticancer agent, the pharmaceutical composition of the present invention, or the cancer chemotherapeutic agent of the present invention as described later. When the concomitant drug is used, the timings of administration of the neutron capture therapy agent of the present invention and the concomitant drug are not limited. The neutron capture therapy agent of the present invention and the concomitant drug may be administered to a subject suffering from a malignant tumor (cancer) at the same time or at different times. When they are administered at different times, the order of administration is not particularly limited.

The contents of the sections "1. Peptide of Present Invention" and "2. Conjugate of Present Invention" all apply to essential matters other than those described above regarding the neutron capture therapy agent of the present invention.

### (Cancer Chemotherapeutic Agent)

The present invention also provides a cancer chemotherapeutic agent including the conjugate of the present invention (hereinafter also referred to as "the cancer chemotherapeutic agent of the present invention"). The conjugate includes the anticancer agent as described above. The cancer chemotherapeutic agent of the present invention can accumulate in a malignant tumor (cancer) expressing the above-described ANXA1 on the cell surface by the action of the peptide of the present invention included in the agent. The accumulation refers to selective orientation (binding) to or localization in a cancerous tissue rather than a non-cancerous tissue (such as a normal tissue) in the subject as described above (such as an animal, more specifically, a mammal (such as a human)). Accordingly, the cancer chemotherapeutic agent of the present invention is suitably used for the treatment of cancer. Thus, the present invention also provides a method for treating or preventing cancer, including administering to a subject an effective dose of the conjugate of the present invention including the anticancer agent or the boron or gadolinium compound as described above, or the like.

Although the content of the conjugate of the present invention in the cancer chemotherapeutic agent of the present invention varies depending on the form of the formulation, the amount of the conjugate of the present invention is typically about 0.01% to 100% by weight, preferably about 0.1% to 50% by weight based on the entire formulation.

The contents of the sections "1. Peptide of Present Invention" and "2. Conjugate of Present Invention" all apply to essential matters other than those described above regarding the cancer chemotherapeutic agent of the present invention.

### (Cancer Testing Reagent)

The present invention also provides a cancer testing reagent including the conjugate of the present invention (hereinafter also referred to as "the cancer testing reagent of the present invention"). The conjugate includes the detectable substance as described above. The cancer testing reagent of the present invention can accumulate in a malignant tumor (cancer) expressing the above-described ANXA1 on the cell surface by the action of the peptide of the present invention included in the agent. The accumulation refers to selective orientation (binding) to or localization in a cancerous tissue rather than a non-cancerous tissue (such as a normal tissue) in the subject as described above (such as an animal, more specifically, a mammal (such as a human)). Accordingly, the cancer testing reagent of the present invention is suitably used for cancer tests. Thus, the present invention also provides a method for detecting cancer, including administering to a subject an effective dose of the conjugate of the present invention including the detectable substance as described above or the like. Cancer tests include radiography, computed tomography (CT), magnetic resonance imaging (MRI), ultrasonography, scintigraphy, positron emission tomography (PET), radionuclide therapy, endoscopy, and laparoscopy as described above. The testing reagent of the present invention may also include a companion diagnostic agent which previously determines the probability that an anticancer agent such as a molecular target drug is effective.

Although the content of the conjugate of the present invention in the cancer testing reagent of the present invention varies depending on the form of the formulation, the amount of the conjugate of the present invention is typically about 0.01% to 100% by weight, preferably about 0.1% to 50% by weight based on the entire formulation.

The contents of the sections "1. Peptide of Present Invention" and "2. Conjugate of Present Invention" all apply to essential matters other than those described above regarding the cancer testing reagent of the present invention.

The present invention will be more specifically described by way of examples below; however, the present invention is by no means limited to these examples.

### Examples

### Example 1: Screening for D-peptide sequence binding to 15 N-terminal residues of annexin A1 within five minutes at 37°C by mirror-image phage display

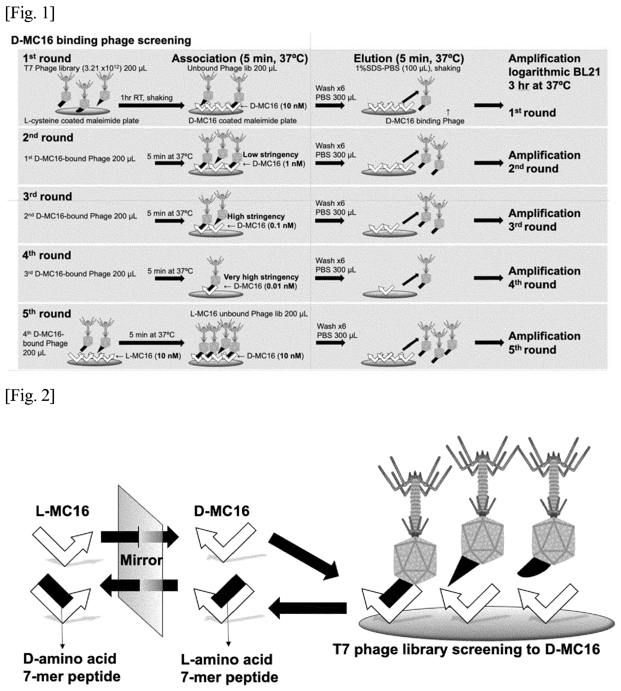

Mirror-image phage display screening was performed to screen for the 7-mer D-peptide sequence selectively binding to the 15 N-terminal residues of annexin A1 (ANXA1) within five minutes at 37°C (FIGS. 1 and 2).

A peptide (D-MC16), in which a d-cys residue was added to the amino acid sequence of the 15 N-terminal residues of ANXA1 synthesized using D-amino acids (mamvseflkqawfie (SEQ ID NO: 29); hereinafter, in the Examples, D-amino acids are represented by small letters), was immobilized on a maleimide-coated plate via the SH group of cys at concentrations shown in FIG. 1 (10 nM, 1 nM, 0.1 nM, and 0.01 nM). 200 µl of a T7 phage mixture displaying a 7-mer L-amino acid peptide library was added to L-cysteine-immobilized wells, and the mixture was stirred and reacted for one hour. 200 µl of the T7 phage mixture not bound to the L-cysteine-immobilized wells was recovered and added to 10 nM D-MC16 peptide-immobilized wells. After maintaining at 37°C for five minutes and washing, the phages bound to the 10 nM D-MC16 peptide were eluted with 100 µl of 1% SDS-PBS (1st output).

The 1st output phage mixture was added to 15 ml of a logarithmic E. coli BL21 culture, and was stirred and cultured at 37°C for three hours. The amplified 1st output phage mixture was obtained as a supernatant after centrifugation. Next, 200 µl of the amplified 1st output phage mixture was added to 1 nM D-MC16 peptide-immobilized wells. After maintaining at 37°C for five minutes and washing, the phages bound to the 1 nM D-MC16 peptide were eluted with 100 µl of 1% SDS-PBS (2nd output). The 2nd output phage mixture was added to 15 ml of a logarithmic E. coli BL21 culture, and was stirred and cultured at 37°C for three hours. The amplified 2nd output phage mixture was obtained as a supernatant after centrifugation.

Next, 200 µl of the 2nd output phage mixture was added to 0.1 nM D-MC16 peptide-immobilized wells. After maintaining at 37°C for five minutes and washing, the phages bound to the 0.1 nM D-MC16 peptide were eluted with 100 µl of 1% SDS-PBS (3rd output). The 3rd output phage mixture was added to 15 mL of a logarithmic E. coli BL21 culture, and was stirred and cultured at 37°C for three hours. The amplified 3rd output phage mixture was obtained as a supernatant after centrifugation. Next, 200 µl of the 3rd output phage mixture was added onto 0.01 nM D-MC16 peptide-immobilized wells. After maintaining at 37°C for five minutes and washing, the phages bound to the 0.01 nM D-MC16 peptide were eluted with 100 µl of 1% SDS-PBS (4th output). The 4th output phage mixture was added to 15 mL of a logarithmic E. coli BL21 culture, and was stirred and cultured at 37°C for three hours. The amplified 4th output phage mixture was obtained as a supernatant after centrifugation.

Next, 200 µl of the 4th output phage mixture was added to 10 nM L-amino acid MC16 peptide (L-MC16)-immobilized wells, and maintained at 37°C for five minutes. 200 µl of the 4th output phage mixture not bound to the L-MC16 was recovered. 200 µl of the 4th output phage mixture not bound to the L-MC16 peptide was added onto 10 nM D-MC16 peptide-immobilized wells. After maintaining at 37°C for five minutes and washing, the phages bound to the 10 nM D-MC16 peptide were eluted with 100 µl of 1% SDS-PBS (5th output).

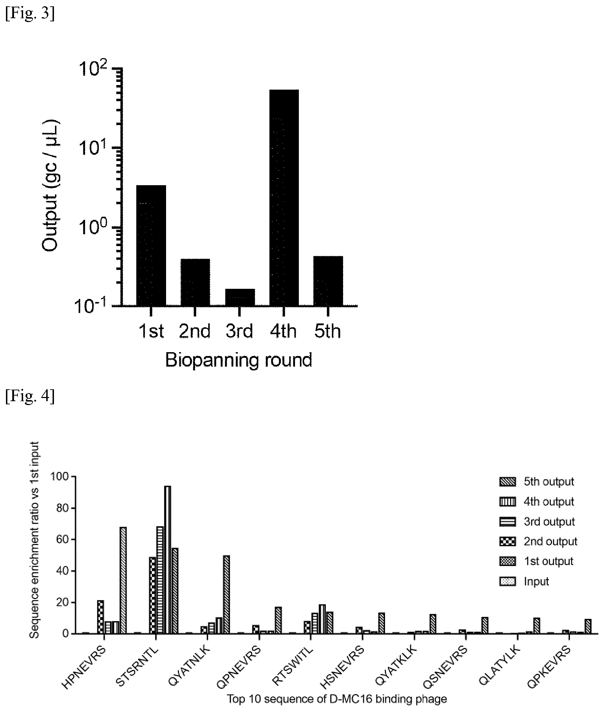

The copy number of phages eluted on each round was calculated by absolute quantification of the genome copy number of T7 phages by digital PCR (FIG. 3). The results of quantification of the copy numbers of the phage mixtures obtained in the 1st to 5th outputs suggested that the copy number of phages bound to D-MC16 was maximized on the fourth round, and that the copy number of phages bound to L-MC16 was subtracted from the copy number of phages bound to D-MC16 and the phages specifically binding to D-MC16 were enriched on the fifth round.

The 7-mer peptide sequences displayed by the phage mixtures obtained in the 1st to 5th outputs were subjected to amplicon sequencing using a next-generation sequencer to analyze the cloning frequency of the peptide sequences displayed by the respective phage mixtures (FIG. 4).

Based on the amplicon sequencing analysis using the next-generation sequencer, HPNEVRS (SEQ ID NO: 30), the most frequent 7-mer peptide sequence in the 5th output, was named HP7 peptide as a dMC16-binding peptide sequence and synthesized as a D-amino acid peptide hpnevrs (dhp7) (SEQ ID NO: 31). STSRNTL (SEQ ID NO: 32) and QYATNLK (SEQ ID NO: 33), the second and third most frequent 7-mer peptide sequences, were also named ST7 and QY7 peptides and synthesized as D-amino acid peptides stsrntl (dst7) (SEQ ID NO: 34) and qyatnlk (dqy7) (SEQ ID NO: 35), respectively. Among half of the top 10 ranked sequences, a common sequence H/Q-P/S-NEVRS was observed (specifically, HPNEVRS (SEQ ID NO: 30), QPNEVRS (SEQ ID NO: 36), HSNEVRS (SEQ ID NO: 37), and QSNEVRS (SEQ ID NO: 38)) (FIG. 5).

### Example 2: Analysis of binding affinity of L-MC16-binding D-peptides for N-terminal MC16 of ANXA1

To measure the binding affinity of the L-MC16-binding D-peptide candidates selected in Example 1 for L-MC16 or D-MC16, biolayer interferometry (BLI) was performed using ForteBio Octet K2 instrument (Sartorius, Gottingen, Germany). It was suggested that the L-MC16 sequence (MAMVSEFLKQAWFIE) (SEQ ID NO: 28) within the N-terminal domain of ANXA1 interacts with dhp7 when L-MC16 is localized in the cell membrane.

In mirror-image phage display screening, the C-terminal side of the displayed L-peptide is exposed to the outermost side of the phage coat protein. Thus, D-peptides (biotin-dhp7, biotin-dst7, and biotin-dqy7) modified with biotin at the N-termini of the candidate D-peptides were synthesized in Biologica Co. 50 µg/ml of each of the biotinylated D-peptides was diluted with a kinetic buffer, and immobilized on Octet Super Streptavidin (SSA) biosensor (Sartorius) so that the C-terminal side of the D-peptide was exposed to the outermost side of the SSA biosensor in a manner similar to peptide display on the phage coat.

As reference, 50 µg/ml of biocytin was immobilized on the SSA biosensor. Each of the immobilized sensors was immersed in a kinetic buffer containing 100 µM, 50 µM, 25 µM, 12.5 µM, 6.25 µM, 3.125 µM, or 1.5625 µM of L-MC16 or D-MC16 for 120 seconds (association phase) and dissociated at 37°C for 120 seconds (dissociation phase), and the biosensor was regenerated.

The biosensor was regenerated by three 5-second pulses using 0.1 mM glycine in hydrochloric acid (pH 2.0). This association-dissociation-regeneration process was performed for an L-MC16 or D-MC16 solution at each concentration. The data were analyzed using Octet Data Analysis HT software, version 10 (Sartorius). The obtained data were fitted to a 1:1 binding model to determine Kon and Koff values and calculate equilibrium dissociation constant KD values (FIGS. 6A to 6C).

The results in FIGS. 6A to 6C showed that among the studied D-peptide sequences, dhp7 having a KD value of 0.48 µM had highest affinity for the L-MC16 sequence, as compared with dqy7 having a KD value of 0.99 µM and dst7 having a KD value of 0.72 µM. It was also found that dhp7 specifically binds to L-MC16, because dhp7 did not bind to D-MC16. Based on these results, dhp7 was used in the subsequent examination.

### Example 3: Analysis of amino acid sequence of L-MC16 critical for binding of dhp7 peptide to N-terminal L-MC16 of L-ANXA1

To identify the L-MC16 amino acids critical for binding of the dhp7 peptide to L-MC16, biolayer interferometry (BLI) was performed using ForteBio Octet K2 instrument (Sartorius, Gottingen, Germany) as in Example 2. 50 µg/ml of biotin-dhp7 was diluted with a kinetic buffer, and immobilized on Octet Super Streptavidin (SSA) biosensor (Sartorius) so that the C-terminal side of dhp7 was exposed to the outermost side of the SSA biosensor in a manner similar to peptide display on the phage coat. As reference, 50 µg/ml of biocytin was immobilized on the SSA biosensor. To evaluate specificity of binding of dhp7 to L-MC16 mutants, binding affinity for 100 µM of the wild type (L-MC16WT) and mutants of L-MC16 was analyzed (FIGS. 7A and 7B).

The results showed that the E6, F7, K9, Q10, W11, F13, and E15 residues of L-MC16 are amino acids critical for binding to dhp7, because binding affinity of dhp7 for L-MC16 mutants E6A, F7A, K9A, Q10A, W11A, F13A, and E15A was remarkably lower than its binding affinity for L-MC16WT (FIGS. 7A and 7B).

### Example 4: Visualization of uptake of dhp7 peptide in ANXA1-positive HEK293 cells by immunofluorescent staining

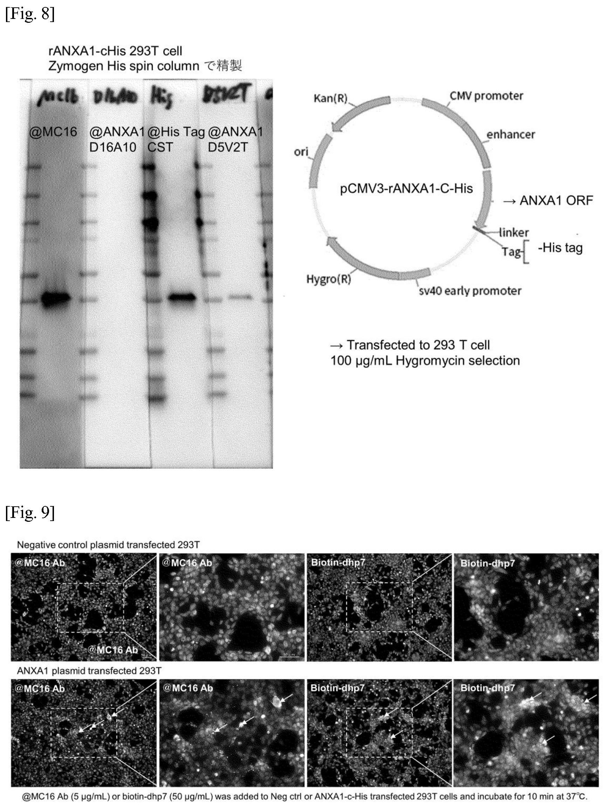

It was examined whether the dhp7 sequence identified in Examples 1 to 3 which selectively binds to the MC16 sequence of ANXA1 can be taken up into ANXA1-positive HEK293 cells. HEK293T cells were transfected with a pCMV3-hANXA1-c-His plasmid into which a ANXA1-c-His fusion gene, in which a His tag was fused to the C-terminus of human ANXA1, was introduced. 72 hours later, the dhp7 peptide biotinylated at the N-terminus (50 µg/ml) or an anti-MC16 antibody reacting with the N-terminus of ANXA1 (5 µg/mL) was added to the HEK293 cells. The cells were incubated at 37°C for 10 minutes, and then fixed with 1% PFA. After adding streptavidin-Alexa Fluor 555 (diluted 500-fold) to detect the biotin-dhp7, or after adding anti-mouse IgG-Alexa Fluor 555 (diluted 500-fold) to detect the anti-MC16 antibody, the cells were incubated at 37°C for 10 minutes and washed with PBS. Then, binding and intracellular uptake of the dph7 peptide in the HEK293 overexpressing Anxa1-c-His were examined by a fluorescence microscope (Keyence BZ9000) (FIGS. 8 and 9).

As a result, the biotin-dhp7 peptide stained the HEK293 overexpressing ANXA1-c-His more intensely than the HEK293 cells into which a negative control plasmid was introduced, exhibited a dotted intracellular staining pattern in a manner similar to ANXA1 detected by the anti-MC16 antibody, and also stained the cytoplasm diffusely. This suggested that dhp7 binds to ANXA1 and is taken up intracellularly.

### Example 5: In-vivo imaging for tumor accumulation of EMCS-Cy7.5-c-hpnevrs peptide

Since it was found in Example 4 that the dhp7 peptide is taken up into ANXA1-positive cells, in-vivo imaging was then attempted by administration of fluorescence-labeled dhp7 to cancer-bearing mice. c(EMCS-Cy7.5)-hpnevrs (Cy7.5-dhp7) (SEQ ID NO: 31) was synthesized, in which a Cy7.5 fluorescent dye is bound to the side chain of the N-terminal d-cys residue of a c-hpnevrs sequence (SEQ ID NO: 31) via an EMCS linker (Formula 3).

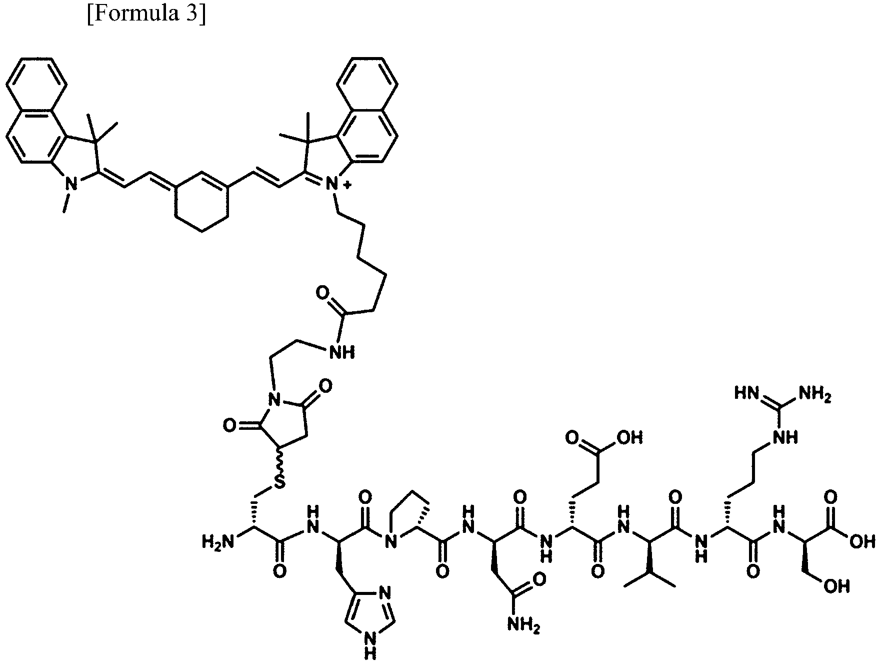

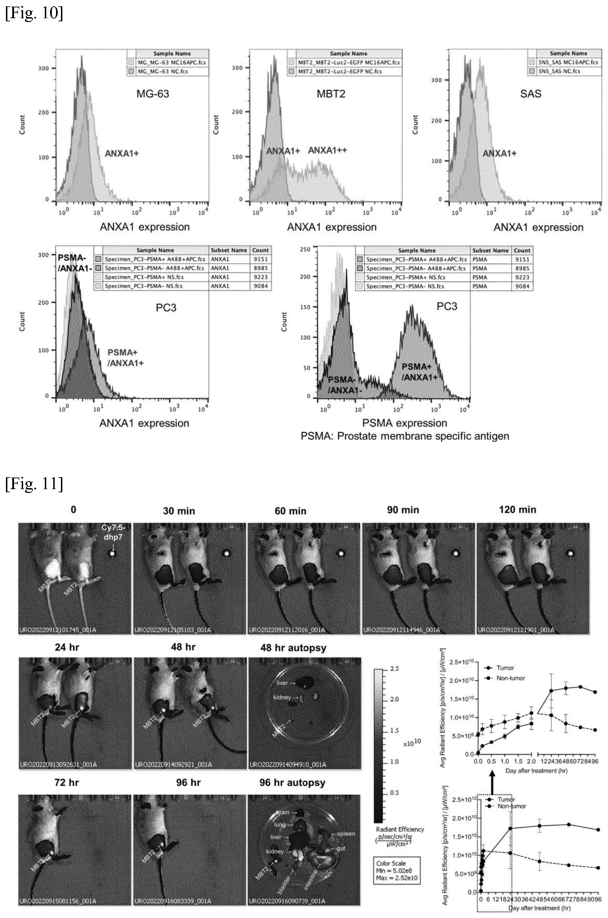

Cellular suspensions of a human head and neck cancer cell line SAS, an osteosarcoma cell line MG-63, a prostate-specific membrane antigen (PSMA)-negative or PSMA-positive prostate cancer cell line PC3-PSMA±, and a mouse bladder cancer cell line MBT2 were reacted with an anti-MC16 antibody reacting with the N-terminus of ANXA1 (1 µg/ml) at room temperature for one hour. The cell pellet was washed and then reacted with an APC-labeled anti-mouse IgG antibody at room temperature for one hour. The cell pellet was washed, and expression of ANXA1 on the cell surface was then examined by flow cytometry. The results are shown in FIG. 10. The antibody reaction was performed without fixation and cell membrane permeation.

The results in FIG. 10 showed that each cancer cell line expresses ANXA1 on the cell surface, although the expression intensity varies by cell line. It was found that the PSMA-negative PC3 (PC3-PSMA-) cell line expresses ANXA1 on the cell surface more weakly than the PSMA-positive PC3 (PC3-PSMA+) cell line does, MG-63 and SAS express ANXA1 on the cell surface comparably, and the MBT2 cells have a cell population that expresses ANXA1 on the cell surface more strongly and a cell population that expresses ANXA1 on the cell surface more weakly.

Tumor accumulation of Cy7.5-c-dhp7 in ANXA1-positive bladder cancer-bearing mice was examined. 4 × 10⁵ mouse bladder cancer cells MBT2 were inoculated into the right hind femur of C3H/HeN mice. One week later, Cy7.5-dhp7 dissolved in physiological saline was intraperitoneally administered at 10 mg/kg (0.2 mg/20 g mouse) to cancer-bearing mice in which the tumor size reached 5 mm. At 0, 30, 90, and 120 minutes and 24, 48, 72, and 96 hours after the administration, the mice were monitored using an in-vivo imaging (IVIS) instrument (FIG. 11).

As a result, Cy7.5-dhp7 exhibited increased fluorescence intensity in the tumor tissue area starting from 30 minutes after the administration, accumulated rapidly until two hours after the administration, reached the plateau at 24 hours after the administration, and maintained strong tumor-specific accumulation until 96 hours after the administration. 48 and 96 hours after the administration, the mice were sacrificed, and the main organs were resected and then imaged ex vivo. As a result, Cy7.5-dhp7 strongly accumulated in the tumor tissue and the kidney, also accumulated in the intestine, but did not accumulate in other organs. This showed that Cy7.5-dhp7 may be renal excretory.

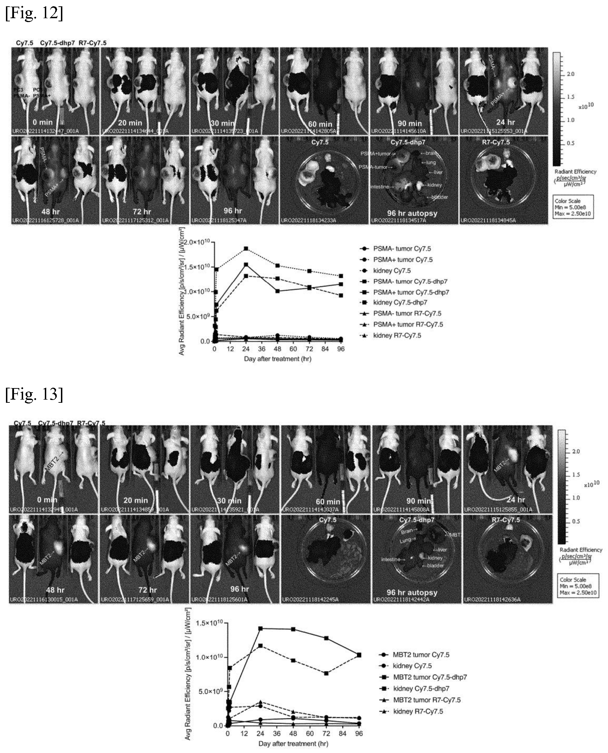

Further, uptake of Cy7.5-dhp7 in other cancer cells was examined. 1 × 10⁶ human prostate cancer cells PC3-PSMA- and PC3-PSMA+, mouse bladder cancer cells MBT2, or human head and neck cancer cells SAS were inoculated into the back of nude mice. Two weeks later, Cy7.5 or Cy7.5-dhp7, or a control L-peptide RRQRRAP(R7)-EMCS-Cy7.5 (SEQ ID NO: 39 (RRQRRAP)) dissolved in physiological saline was intraperitoneally administered at 10 mg/kg (0.2 mg/20 g mouse) to each cancer-bearing mouse in which the tumor size reached 15 mm. At 0, 20, 30, 90, and 120 minutes and 24, 48, 72, and 96 hours after the administration, the mice were monitored using an in-vivo imaging (IVIS) instrument (FIGS. 12 to 14).

As a result, Cy7.5-dhp7 reached the maximum at 24 hours after the administration and then gradually reduced its tumor accumulation in each cancer-bearing nude mouse. 96 hours after the administration, the mice were sacrificed, and the main organs were resected and then imaged ex vivo. The results showed that Cy7.5-dhp7 may be renal excretory, because Cy7.5-dhp7 strongly accumulated in the tumor tissue, the kidney, and the intestine and did not accumulate in other organs. It was found that dhp7 has tumor specificity to prostate cancer, bladder cancer, and head and neck cancer, because Cy7.5 alone and the control L-R7 peptide-Cy7.5 did not exhibit tumor-specific accumulation.

### Example 6: Visualization of uptake of EMCS-¹⁰BSH-dhp7 peptide in ANXA1-positive cancer cells by immunofluorescent staining

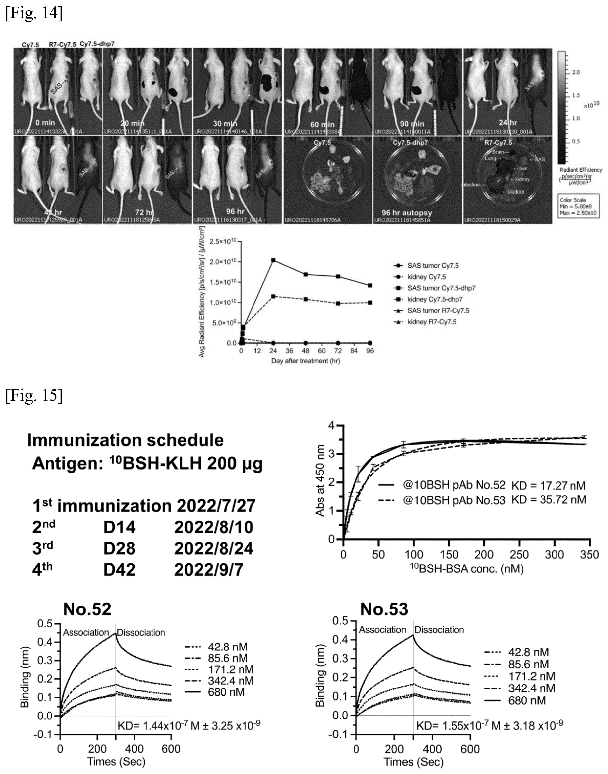

Since it was found in Example 5 that Cy7.5-dhp7 is taken up into ANXA1-positive tumor tissues in cancer-bearing mouse models, uptake of EMCS-¹⁰BSH-dhp7 (¹⁰BSH-dhp7) in various ANXA1-positive cancer cells was then examined. To confirm whether ¹⁰BSH is taken up into the cancer cells examined in Example 5, rabbit anti-¹⁰BSH polyclonal antibodies specifically reacting with ¹⁰BSH were prepared. Two mice were immunized with 200 µg of ¹⁰BSH-KLH as an antigen. After the fourth booster inoculation, the whole blood was collected to obtain sera (#52 and #53) containing rabbit anti-¹⁰BSH polyclonal antibodies specifically reacting with ¹⁰BSH. An IgG fraction was purified from each serum in Protein A column. Affinity of the rabbit anti-¹⁰BSH polyclonal antibodies (#52 and #53) for ¹⁰BSH-BSA was analyzed by ELISA and BLI. The results are shown in FIG. 15.

The results in FIG. 15 confirmed that both rabbit anti-¹⁰BSH polyclonal antibodies (#52 and #53) react ¹⁰BSH-BSA concentration-dependently. In the following experiment, the #52 or #53 antibody was used for detecting ¹⁰BSH.

Next, EMCS-¹⁰BSH-hpnevrs (¹⁰BSH-dhp7) (SEQ ID NO: 31) was synthesized, in which ¹⁰BSH is bound to the side chain of the N-terminal His residue of an hpnevrs (dhp7) (SEQ ID NO: 31) sequence via an EMCS linker (Formula 4).

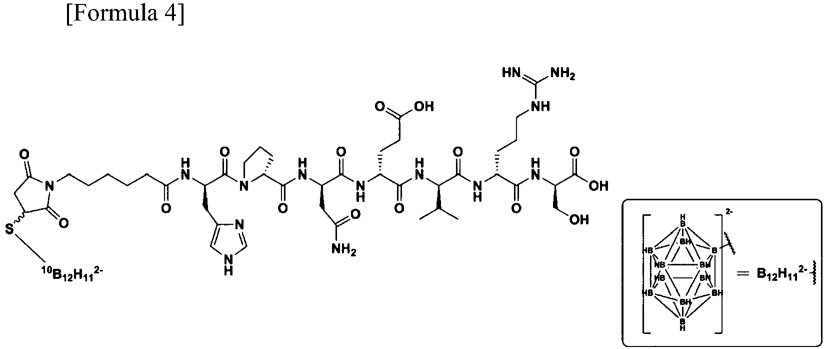

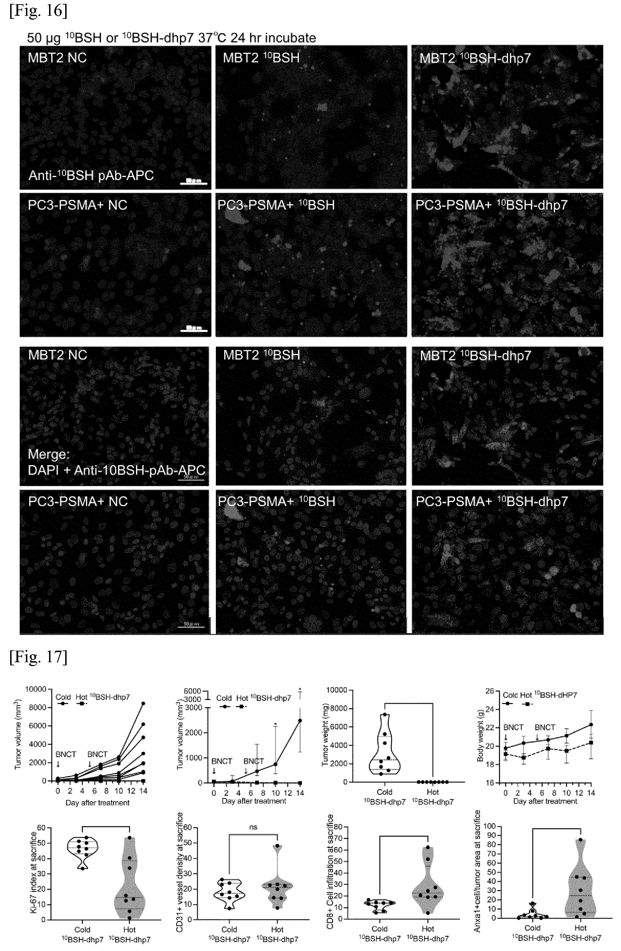

Uptake of ¹⁰BSH-dhp7 in cancer cells was examined. 50 µg of ¹⁰BSH-dhp7 or ¹⁰BSH was added to MBT2 and PC3-PSMA+ cells seeded in a 3.5 cm culture dish (ib81156) manufactured by ibidi. 24 hours later, the cells were washed with PBS three times and then fixed with 4% PFA. After blocking with 3% BSA BD PhosFlow perm/wash buffer I (BD557885), 3% BSA BD PhosFlow perm/wash buffer I containing 1 µg/ml of the rabbit anti-¹⁰BSH polyclonal antibody #52 was reacted at room temperature for one hour with ¹⁰BSH taken up into the cells. After washing with PBS three times, it was reacted at room temperature for one hour with an APC-labeled anti-mouse IgG antibody (diluted 1000-fold) to detect the rabbit anti-¹⁰BSH polyclonal antibody, and washed with PBS three times. Then, intracellular uptake of ¹⁰BSH-dhp7 or ¹⁰BSH in each cancer cell was examined by a fluorescence microscope (Keyence BZ9000) (FIG. 16).

As a result, dotted intracellular fluorescence was observed in each cancer cell to which ¹⁰BSH-dhp7 was added, as compared with each cancer cell to which ¹⁰BSH was added. It was found that more ¹⁰BSH is taken up intracellularly by ¹⁰BSH-dhp7 at a 5.44-fold lower concentration, because ¹⁰BSH has a molecular weight of 219.87 and ¹⁰BSH-dhp7 has a molecular weight of 1197.5. It was found that the efficiency of intracellular uptake of ¹⁰BSH is enhanced via ANXA1 on the cell surface by binding to the dhp7 peptide, because ¹⁰BSH has low cell membrane permeability.

### Example 7: Boron neutron capture therapy by EMCS-¹⁰BSH-hpnevrs (¹⁰BSH-dhp7)

Since it was found in Example 6 that ¹⁰BSH-dhp7 is taken up into Anxa1-positive cancer cells, boron neutron capture therapy by ¹⁰BSH-dhp7 was attempted in a mouse bladder cancer model. 2 × 10⁵ mouse bladder cancer cells MBT2 were inoculated into the right hind femur of C3H/HeN mice. Two weeks later, EMCS-¹⁰BSH-dhp7 dissolved in physiological saline was intraperitoneally administered at 20 mg/kg (0.4 mg/20 g mouse) to cancer-bearing mice in which the tumor size reached 5 mm.

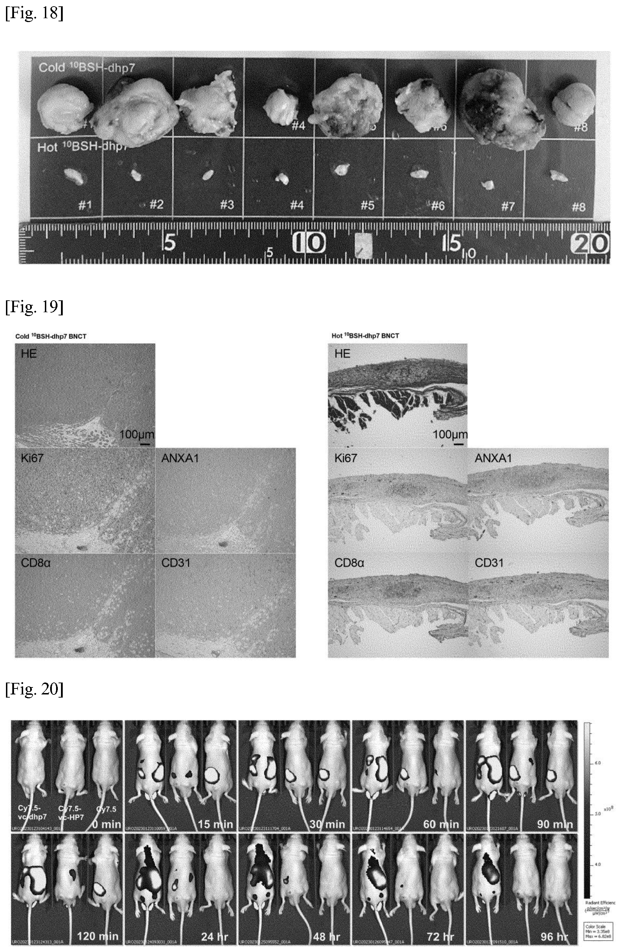

Starting from 48 hours after the administration, the tumor area was irradiated with thermal neutrons at 20 MeV 100 mA for 60 minutes using an accelerator manufactured by Sumitomo Heavy Industries (dose: 1.63 × 10¹²/cm², average 0.23 Gy). One week after the irradiation, second BNCT treatment was performed. 24 hours prior to irradiation with thermal neutrons, 20 mg/kg (0.4 mg/20 g mouse) of ¹⁰BSH-dhp7 was intraperitoneally administered. Irradiation with thermal neutrons was then performed. One week after the second irradiation, mice of each group were sacrificed. The tumor size and the tumor weight were measured, and pathological analysis of the resected tissue was performed to verify the antitumor effect. The tumor volume was measured from the start of administration until sacrifice by the calculation formula: V = long axis × short axis × short axis/2) (FIG. 17). As a result, no tumor reduction effect was observed in the ¹⁰BSH-dhp7 administration and non-BNCT treatment group, while a remarkable tumor reduction effect was observed immediately after the first BNCT and tumors almost completely disappeared one week after the second irradiation in the ¹⁰BSH-dhp7 administration and BNCT treatment group (FIG. 18).

The results of pathological analysis of the resected tissues are shown in FIG. 19. The results showed that there are no residual tumors in four of eight cases in the ¹⁰BSH-dhp7 administration and BNCT treatment group. The results also showed that marked intratumoral infiltration was observed in CD8α-positive immune cells, and expression of ANXA1 was markedly enhanced in the residual tumor cells, in the remaining four cases. ¹⁰BPA currently used in actual clinical practice requires intravenous bolus administration at 500 mg/kg for two to three hours in order to achieve the therapeutic effect of BNCT. This poses a greater burden on patients and also limits the treatment time. ¹⁰BSH-dhp7 is believed to be a drug that poses a reduced burden on patients and allows greater flexibility in treatment timing as compared with a conventional drug, because boron continues to accumulate in the tumor site until 2 to 24 hours after administration at a dose 25-fold lower than that of a conventional drug.

### Example 8: In-vivo imaging for tumor accumulation of c(MI-Cy7.5)-vc-hpnevrs peptide

Although tumor accumulation of the EMCS-Cy7.5-c-hpnevrs peptide (SEQ ID NO: 31) in cancer-bearing mouse models was examined in Example 5, the fluorescence-labeled peptides shown below were synthesized to examine tumor accumulation when using another linker instead of the EMCS linker.

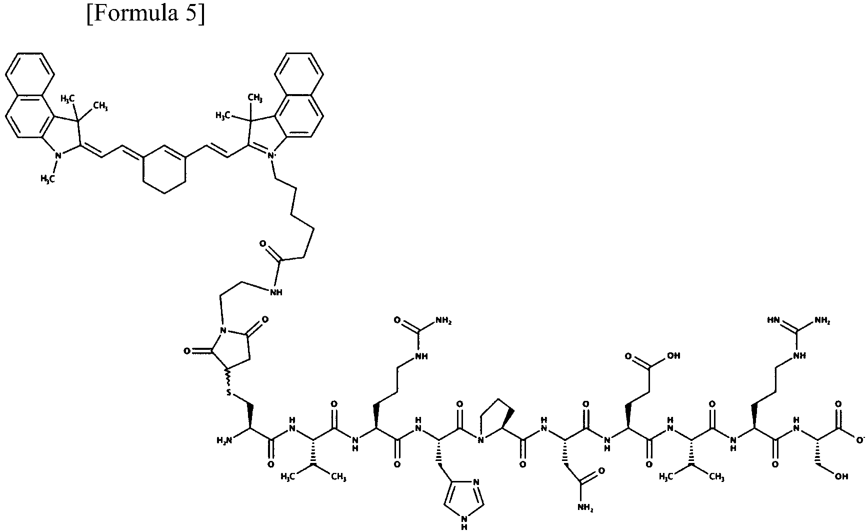

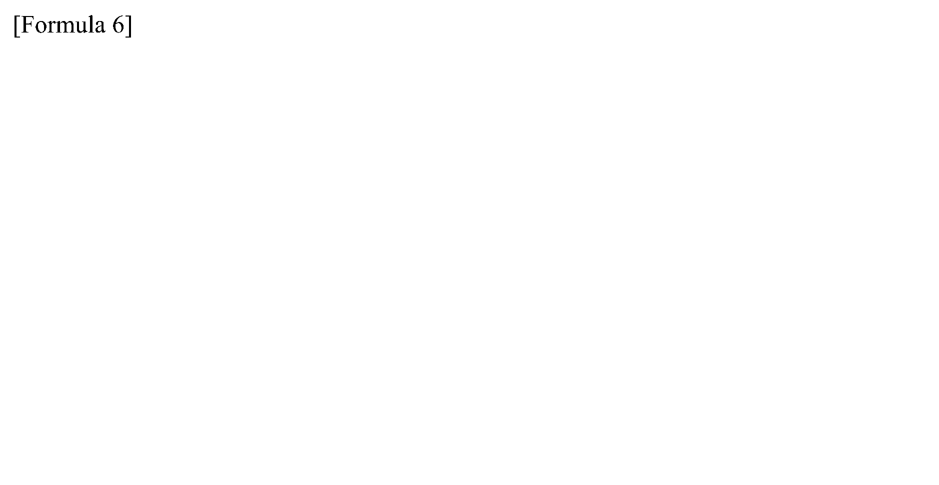

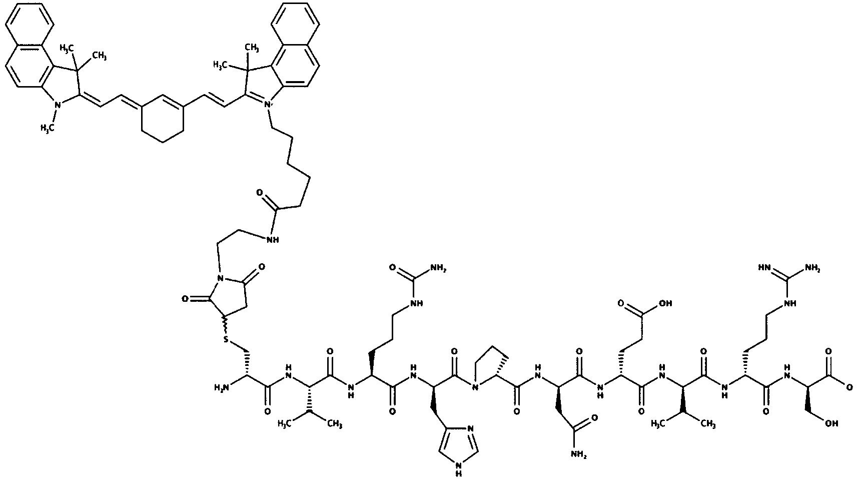

C(MI-Cy7.5)-vc-HPNEVRS (Cy7.5-vc-LHP7) (SEQ ID NO: 30 (HPNEVRS)) was synthesized, in which a Cy7.5 fluorescent dye is bound via a Val-Cit linker to the side chain of the N-terminal Cys residue of a C-HPNEVRS sequence (SEQ ID NO: 30) to which a L-Cys residue is added (Formula 5). c(MI-Cy7.5)-vc-hpnevrs (Cy7.5-vc-dhp7) (SEQ ID NO: 31 (hpnevrs)) was synthesized, in which a Cy7.5 fluorescent dye is bound via a Val-Cit linker to the side chain of the N-terminal Cys residue of a c-hpnevrs sequence (SEQ ID NO: 31) to which a d-Cys residue is added (Formula 6).

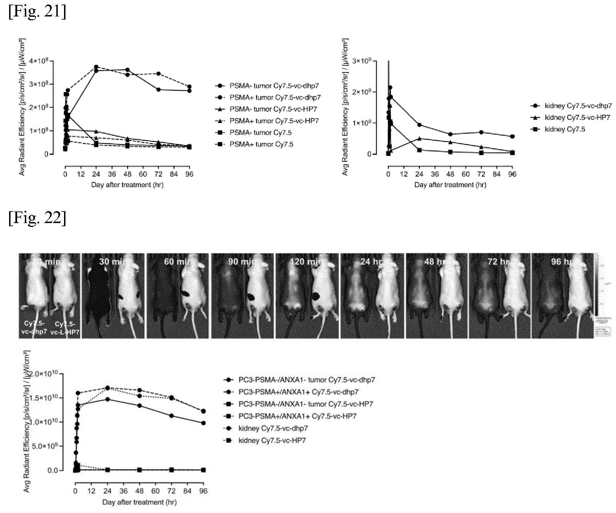

Next, 1 × 10⁶ human prostate cancer cells PC3-PSMA-/ANXA1- and PC3-PSMA+/ANXA1+ were inoculated into the back of nude mice. One week later, 10 mg/kg (0.2 mg/20 g mouse) of Cy7.5, 28.7 mg/kg (0.574 mg/20 g mouse) of Cy7.5-vc-dhp7, or 28.7 mg/kg (0.574 mg/20 g mouse) of Cy7.5-vc-LHP7 (L-HP7 peptide) dissolved in physiological saline was intraperitoneally administered to each cancer-bearing mouse in which the tumor size reached 7 mm. Each fluorescent compound was prepared to provide a dose corresponding to a Cy7.5 concentration of 10 mg/kg (0.2 mg/20 g mouse). At 0, 15, 30, 60, 90, and 120 minutes and 24, 48, 72, and 96 hours after the administration, the mice were monitored using an in-vivo imaging (IVIS) instrument (FIGS. 20 and 21).

As a result, Cy7.5-vc-dhp7 accumulated in the tumor tissue selectively, regardless of the expression of ANXA1 in the tumor, reached the maximum at 24 hours after the administration, and then gradually reduced accumulation, but maintained accumulation until 96 hours after the administration. Meanwhile, administration of Cy7.5-vc-LHP7 and Cy7.5 caused no accumulation in the tumor. This suggested that dhp7, a D-amino acid peptide, may selectively bind to and deliver a fluorescent dye to ANXA1 expressed in the tumor vessel or tumor. It was found to be critical for any sequence to be constituted by D-amino acids to exhibit tumor-targeting activity. It was also suggested that each of the drugs is renal excretory, because it accumulated in the kidney strongly.

Further, variation in accumulation depending on the tumor size was examined. 28.7 mg/kg (0.574 mg/20 g mouse) of Cy7.5-ve-dhp7 or 28.7 mg/kg (0.574 mg/20 g mouse) of Cy7.5-vc-LHP7 (L-HP7 peptide) dissolved in physiological saline was intraperitoneally administered to each cancer-bearing mouse in which the tumor size reached 15 mm. Tumor accumulation was examined in the same manner as above (FIG. 22).

As a result, Cy7.5-ve-dhp7 began to accumulate in the tumor tissue selectively at 60 minutes after the administration, regardless of the expression of ANXA1 in the tumor, reached the maximum at 2 to 24 hours after the administration, and then gradually reduced accumulation, but maintained accumulation until 96 hours after the administration. Meanwhile, it was suggested that dhp7 constituted by D-amino acids may accumulate in the tumor tissue more rapidly as the tumor size is larger, because no Cy7.5-vc-LHP7 accumulated in the tumor.

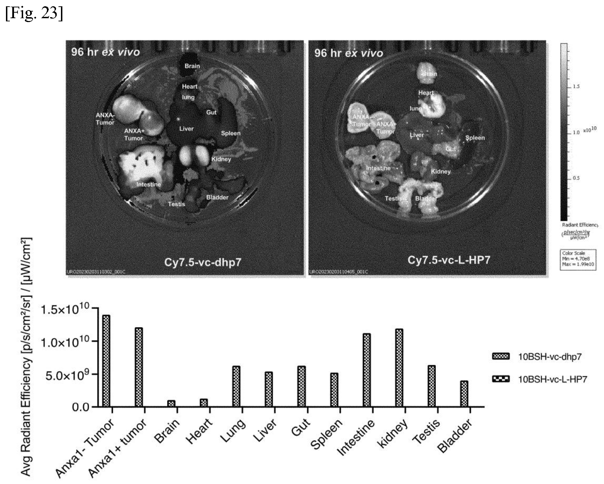

96 hours after the administration, the mice were sacrificed, and the main organs were resected and then imaged ex vivo. The results are shown in FIG. 23. The results in FIG. 23 showed that Cy7.5-vc-dhp7 may be renal excretory, because Cy7.5-vc-dhp7 strongly accumulated in the tumor tissue, the kidney, and the intestine and weakly accumulated in other organs. It was found that dhp7 has tumor specificity to prostate cancer, because Cy7.5-vc-LHP7 did not exhibit tumor-specific accumulation.

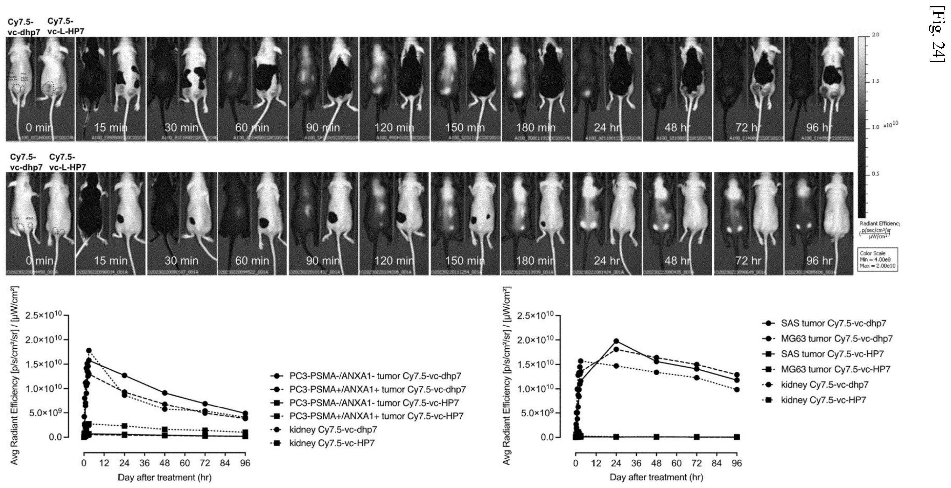

Since Cy7.5-vc-dhp7 was shown to have tumor-specific accumulation in the prostate cancer mouse model, tumor accumulation of Cy7.5-ve-dhp7 in cancer cells other than prostate cancer cells (human head and neck cancer cells SAS and human osteosarcoma cancer cells MG-63) was examined under the same conditions as in FIG. 17 (tumor size: 7 mm) (FIG. 24). As a result, Cy7.5-ve-dhp7 began to accumulate in the tumor tissue selectively at 60 minutes after the administration, regardless of the expression of ANXA1 in the tumor, reached the maximum at 2 to 24 hours after the administration, and then gradually reduced accumulation, but maintained accumulation until 96 hours after the administration. Meanwhile, it was shown that dhp7 constituted by D-amino acids accumulates in the tumor tissue in a manner similar to the prostate cancer model, because no Cy7.5-vc-LHP7 accumulated in the tumor.

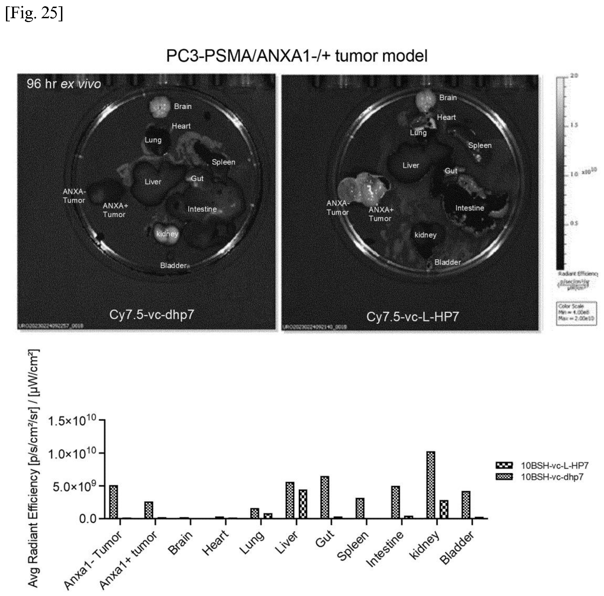

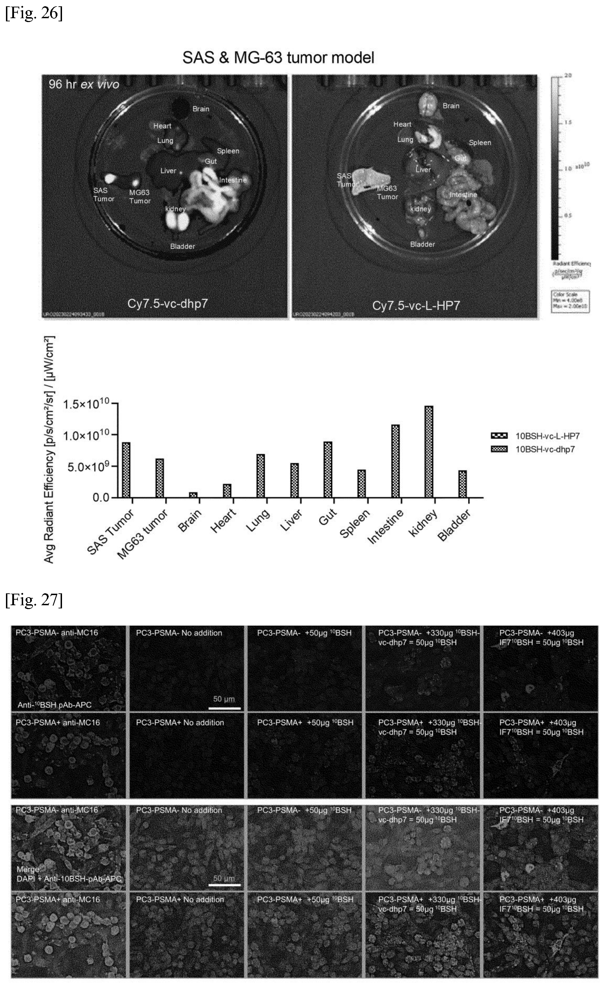

96 hours after the administration, the mice were sacrificed, and the main organs were resected and then imaged ex vivo. The results are shown in FIGS. 25 and 26. The results in FIGS. 25 and 26 showed that Cy7.5-vc-dhp7 may be renal excretory, because Cy7.5-vc-dhp7 strongly accumulated in the tumor tissue, the kidney, and the intestine and weakly accumulated in other organs. It was found that dhp7 has tumor specificity to head and neck cancer and osteosarcoma, because Cy7.5-ve-LHP7 did not exhibit tumor-specific accumulation.

### Example 9: Visualization of uptake of EMCS-¹⁰BSH-vc-dhp7 peptide in ANXA1-positive cancer cells by immunofluorescent staining

Since it was found in Example 8 that Cy7.5-vc-dhp7 is taken up into ANXA1-positive cancer cell mouse models, EMCS-¹⁰BSH-val-cit-hpnevrs (¹⁰BSH-vc-dhp7) (SEQ ID NO: 31 (hpnevrs)) (Formula 7) was then synthesized, in which EMCS-¹⁰BSH is bound to the N-terminus of an hpnevrs (dhp7) sequence (SEQ ID NO: 31) via a Val-Cit linker, and its uptake in ANXA1-positive cancer cells was examined.

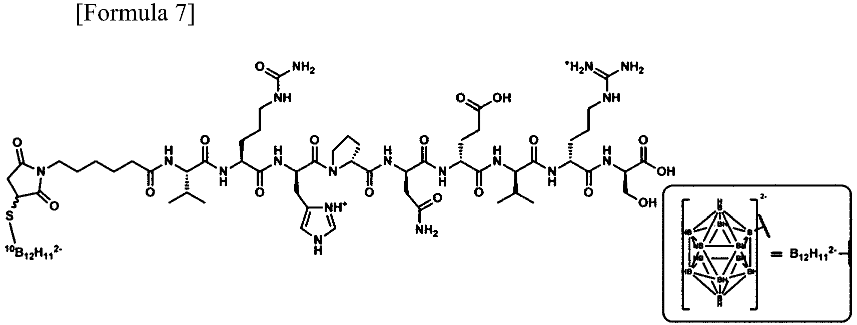

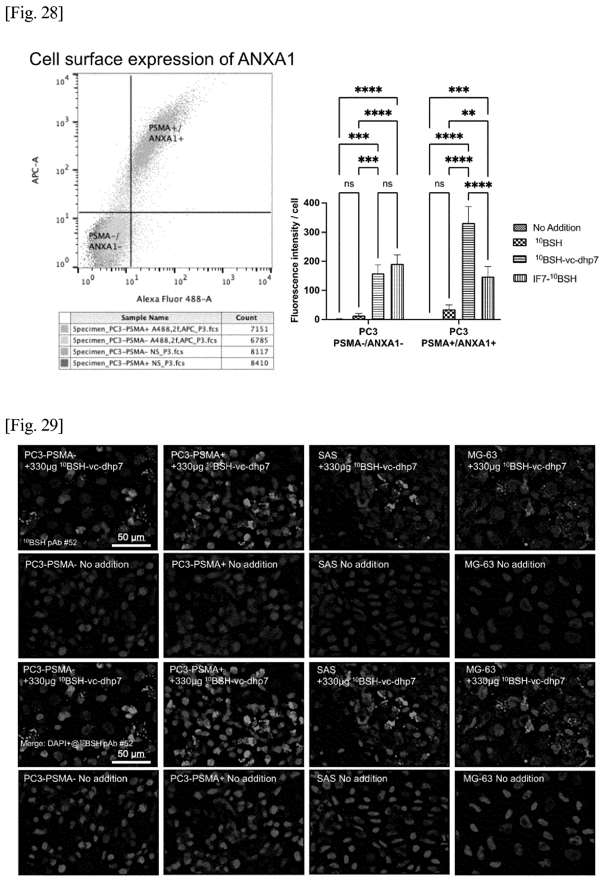

Uptake of ¹⁰BSH-vc-dhp7 in cancer cells was examined. 330 µg of ¹⁰BSH-vc-dhp7, 403.6 µg of IFLLWQRK(EMCS-¹⁰BSH)RR (IF7-¹⁰BSH (a compound described in WO2019244954)) (SEQ ID NO: 40 (IFLLWQRK)), or 50 µg of ¹⁰BSH was added to PC3-PSMA+ and PC3-PSMA- cells seeded in a 3.5 cm culture dish (ib81156) manufactured by ibidi. 24 hours later, the cells were washed with PBS three times and then fixed with 4% PFA. Each peptide-boron compound was prepared to have a boron concentration that allows 50 µg of ¹⁰BSH to be finally added. After blocking with 3% BSA BD PhosFlow perm/wash buffer I (BD557885), 3% BSA BD PhosFlow perm/wash buffer I containing 1 µg/ml of the rabbit anti-¹⁰BSH polyclonal antibody #52 was reacted at room temperature for one hour with ¹⁰BSH taken up into the cells. After washing with PBS three times, it was reacted at room temperature for one hour with an APC-labeled anti-mouse IgG antibody (diluted 1000-fold) to detect the rabbit anti-¹⁰BSH polyclonal antibody, and washed with PBS three times. Then, intracellular uptake of ¹⁹BSH-vc-dhp7, IF7-¹⁰BSH, or ¹⁰BSH in each cancer cell was examined by a fluorescence microscope (Keyence BZ9000) (FIGS. 27 and 28).

As a result, more dotted intracellular fluorescence was observed in each cancer cell to which ¹⁰BSH-vc-dhp7 was added, as compared with each cancer cell to which ¹⁰BSH was added. The fluorescence intensity per cell was quantified to show that the uptake efficiency was significantly high. This examination showed that more ¹⁰BSH was taken up into cells to which ¹⁰BSH-vc-dhp7 was added, because the drugs were each prepared to include 50 µg of ¹⁰BSH. It was found that the efficiency of intracellular uptake of ¹⁰BSH is enhanced via ANXA1 on the cell surface by binding to the dhp7 peptide, because ¹⁰BSH has low cell membrane permeability. It was also suggested that expression of ANXA1 on the cell surface may influence the efficiency in uptake via the dhp7 peptide, because the PSMA-negative cells expressing less ANXA1 on the cell surface (FIGS. 25 and 26) took up ¹⁰BSH-vc-dhp7 less than the PSMA-positive cells did. It was found that in the PSMA-positive cells highly expressing ANXA1, the efficiency in uptake of ¹⁰BSH in the ¹⁰BSH-vc-dhp7 addition group was 9.5 to 11.7-fold significantly higher than that in the BSH alone or IF7-¹⁰BSH (a compound described in WO2019244954) group.

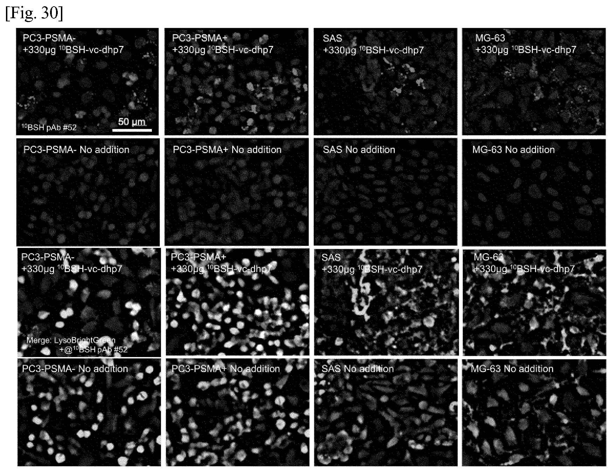

The results in FIGS. 27 and 28 showed that the efficiency of intracellular intake of ¹⁰BSH in ANXA1-positive PC3 cells was statistically significantly higher in the ¹⁰BSH-vc-dhp7 treatment group than in the IF7-¹⁰BSH and ¹⁰BSH treatment groups. Thus, the efficiency in intracellular uptake of ¹⁰BSH in cancer cells other than PC3 cells (human head and neck cancer cells SAS and human osteosarcoma cancer cells MG-63) was examined under the same conditions as in FIGS. 27 and 28. The results are shown in FIGS. 29 to 31.

The results of FIGS. 29 to 31 showed statistically significant intracellular intake of ¹⁰BSH in all cells of the ¹⁰BSH-vc-dhp7 addition group, as compared with the ¹⁰BSH-vc-dhp7 non-addition group. Among the studied cell lines, the uptake efficiency was highest in PC3-PSMA+ cells expressing most ANXA1 on the cell surface. It was shown that SAS, MG63, and PC3-PSMA- expressing ANXA1 weakly positively on the cell surface exhibited similar ¹⁰BSH uptake efficiencies, although such efficiencies varied by cell line. It was also shown that ¹⁰BSH taken up into each cell was colocalized with lysosome stained with LysoBright Green. This suggested that ¹⁰BSH-vc-dhp7 may be taken up into each cell by endocytosis via ANXA1 expressed on the cell surface, the Val-Cit linker may be cleaved by cathepsin B in lysosome, and 10BSH may be released into the cell (FIGS. 29 to 31).

### Example 10: Examination on tumor boron accumulation for ¹⁰BSH-vc-dhp7 peptide in cancer-bearing mice

It was found in Example 9 that EMCS-¹⁰BSH-vc-dhp7 is taken up into Anxa1-positive cancer cells. Thus, immunohistochemical staining using an anti-¹⁰BSH antibody was performed to examine whether the intratissue ¹⁰B accumulation concentrations in tumor, normal kidney, and femur muscle tissues in a human prostate cancer model reached ¹⁰B concentrations sufficient for exhibiting the antitumor effect of neutron capture therapy. 1 × 10⁶ human prostate cancer cells PC3-PSMA-/ANXA1- were inoculated into the left back of nude mice, and 1 × 10⁶ human prostate cancer cells PC3-PSMA+/ANXA1+ were inoculated into the right back of the nude mice. Two weeks later, 50 mg/kg (1.25 mg/25 g mouse) of ¹⁰BSH, 330 mg/kg (8.25 mg/25 g mouse) of EMCS-¹⁰BSH-vc-dhp7, or 403.5 mg/kg (10.08 mg/25 g mouse) of IFLLWQRK(EMCS-¹⁰BSH)RR (IF7-¹⁰BSH (a compound described in WO2019244954)) (SEQ ID NO: 40 (IFLLWQRK)) dissolved in physiological saline was intraperitoneally administered to each cancer-bearing mouse in which the tumor size reached 10 mm.

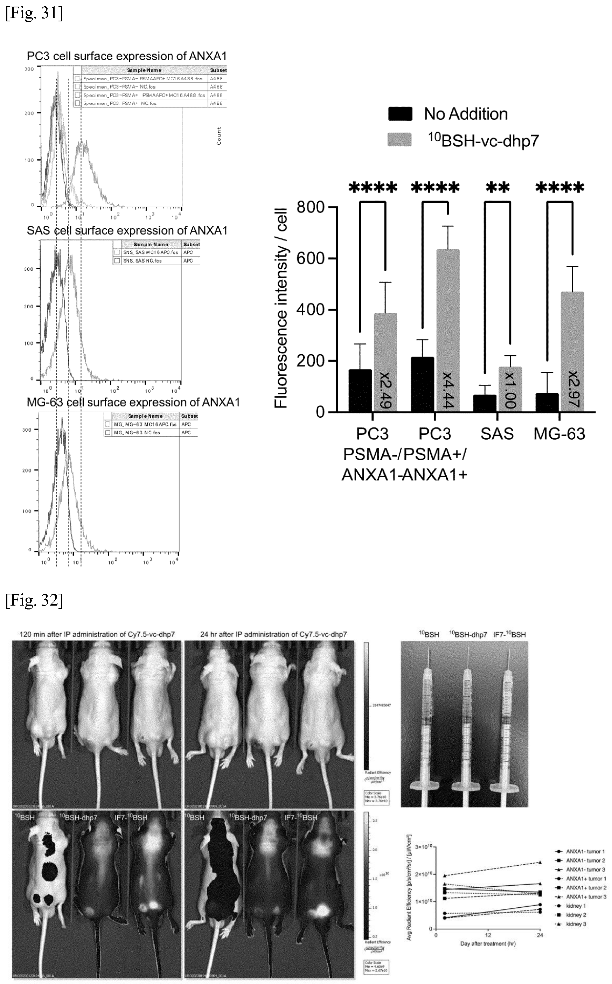

¹⁰BSH alone and ¹⁰BSH-vc-dhp7 were in a dissolved state in physiological saline at such concentrations, while IF7-¹⁰BSH was poorly soluble and cloudy at such a concentration. Thus, it was shown that ¹⁰BSH-vc-dhp7 is more soluble and more flexible in designing the dosage form than IF7-¹⁰BSH. 0.574 mg/kg of Cy7.5-vc-dhp7 was administered to each mouse at the same time to confirm that dhp7 accumulated (FIG. 32).

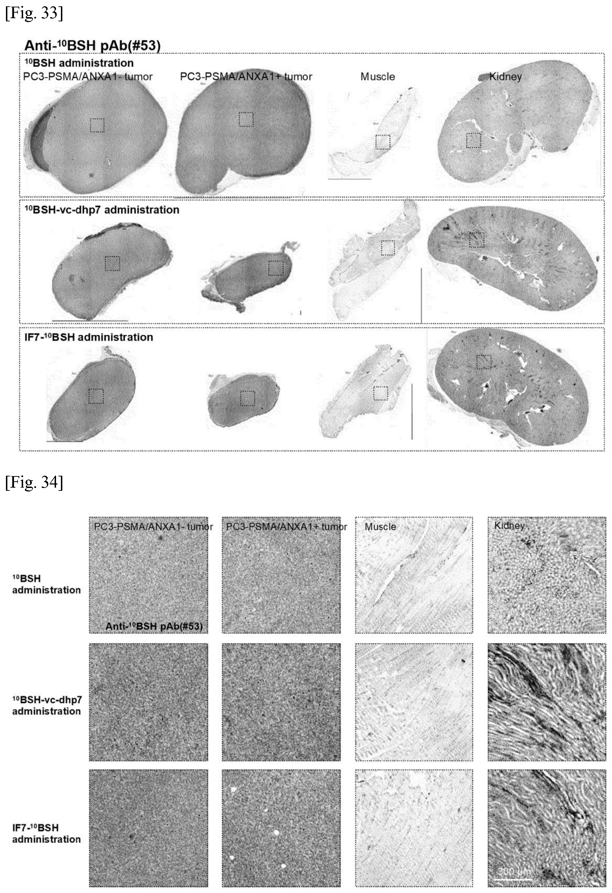

Cy7.5-ve-dhp7 began to increasingly accumulate in the tumor tissue area at 120 minutes after the administration, and strongly accumulated in the tumor area and the kidney at 24 hours after the administration when the mouse was sacrificed (FIG. 32). The tumor tissue and the kidney in which Cy7.5-ve-dhp7 strongly accumulated and the femur muscle tissue in which Cy7.5-ve-dhp7 weakly accumulated were resected, respectively, and fixed with formalin and embedded in paraffin to prepare tissue sections. Each tissue section was immunohistochemically stained with an immunostaining anti-¹⁰BSH antibody (#53, 1 µg/ml). The results are shown in FIGS. 33 to 35.

The results in FIGS. 33 to 35 showed that the tumor and kidney tissues in the ¹⁰BSH-vc-dhp7 administration group were stained more strongly than those in the ¹⁰BSH administration group and the IF7-¹⁰BSH administration group.

The above examination showed that EMCS-¹⁰BSH-vc-dhp7 is taken up into Anxa1-positive cancer cells and excreted from the kidney. Thus, ¹⁰B accumulation in a human prostate cancer model was visualized with an AlexaFluor 750-labeled anti-¹⁰BSH antibody, and the in vivo distribution was examined by IVIS. 1 × 10⁶ human prostate cancer cells PC3-PSMA-/ANXA1- were inoculated into the left femur of nude mice, and 1 × 10⁶ human prostate cancer cells PC3-PSMA+/ANXA1+ were inoculated into the right femur of nude mice. Two weeks later, 330 mg/kg (8.25 mg/25 g mouse) of ¹⁰BSH-vc-dhp7 dissolved in physiological saline was intraperitoneally administered to each cancer-bearing mouse in which the tumor size reached 10 mm. ¹⁰BSH-vc-dhp7 was in a dissolved state in physiological saline at such a concentration. 0.05 mg/kg of an AlexaFluor750-labeled anti-¹⁰BSH antibody (#52) was intraperitoneally administered to each mouse at the same time to confirm that ¹⁰BSH accumulated.

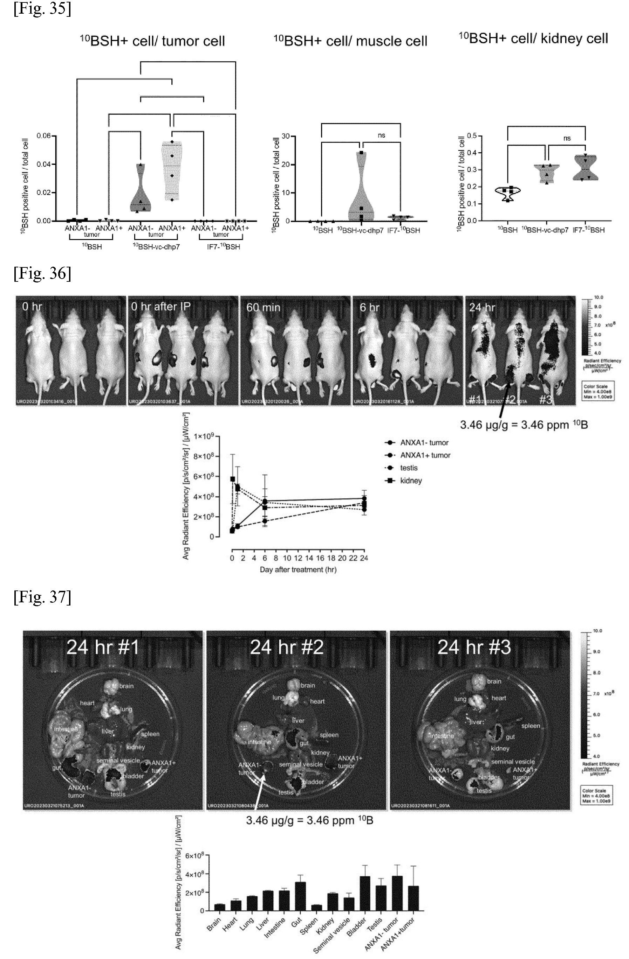

As a result, ¹⁰BSH accumulated near the kidney and the testis immediately after the intraperitoneal administration, as in the case of Cy7.5-vc-dhp7 (FIG. 36). At 24 hours after the administration, accumulation in the kidney disappeared, and strong accumulation in the tumor site was observed, regardless of the expression of ANXA1 (FIG. 36). The results of ex vivo imaging of the resected organs at 24 hours after the administration are shown in FIG. 37.

The results showed that ¹⁰BSH selectively accumulates in the tumor site by ¹⁰BSH-vc-dhp7, because strong accumulation in the tumor site was observed in each mouse at 24 hours after the administration.

### Example 11: Boron neutron capture therapy by ¹⁰BSH-val-cit-dhp7 (EMCS-¹⁰BSH-vc-dhp7)

Since it was found in Example 10 that the boron concentration in the tumor tissue by administration of ¹⁰BSH-vc-dhp7 is sufficient for the antitumor effect of neutron capture therapy (BNCT), boron neutron capture therapy by EMCS-¹⁰BSH-vc-dhp7 was attempted in ANXA1-negative human prostate cancer and ANXA 1-positive head and neck cancer models. 1 × 10⁶ human prostate cancer cells PC3-PSMA-/ANXA1- or human head and neck cancer cells SAS were inoculated into the right hind femur of nude mice. One week later, 50 mg/kg (1.25 mg/25 g mouse) of ¹⁰BSH or 330 mg/kg (8.25 mg/25 g mouse) of ¹⁰BSH-vc-dhp7 dissolved in physiological saline was intraperitoneally administered to each cancer-bearing mouse in which the tumor size reached 5 mm. Starting from 24 hours after the administration, the tumor area was irradiated with thermal neutrons at 20 MeV 100 mA for 60 minutes using an accelerator manufactured by Sumitomo Heavy Industries (dose: 1.63 × 10¹²/cm², average 0.23 Gy). One week after the irradiation, second BNCT treatment was performed.

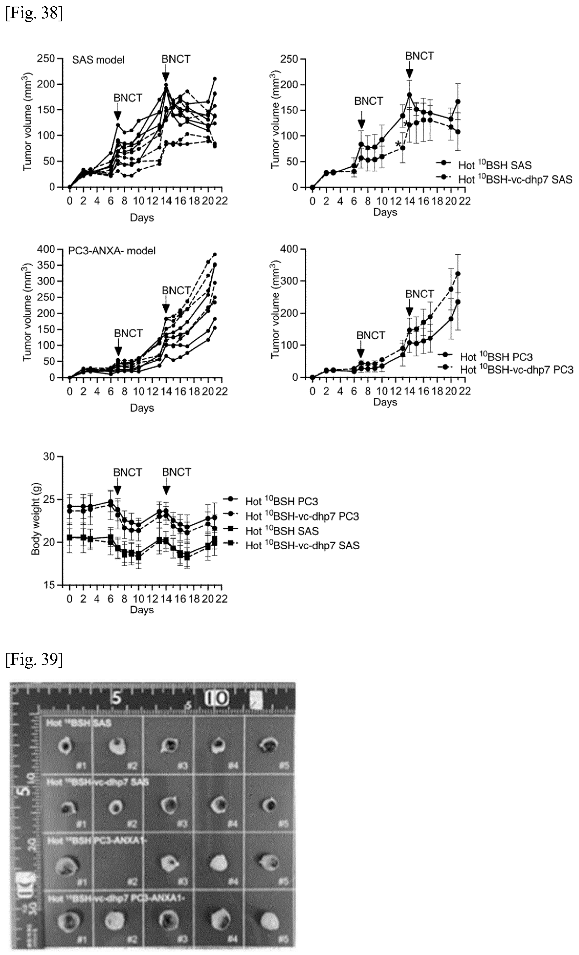

24 hours prior to irradiation with thermal neutrons, 50 mg/kg (1.25 mg/25 g mouse) of ¹⁰BSH or 330 mg/kg (8.25 mg/25 g mouse) of ¹⁰BSH-vc-dhp7 dissolved in physiological saline was intraperitoneally administered to each mouse. Irradiation with thermal neutrons was then performed. One week after the second irradiation, mice of each group were sacrificed. The tumor size and the tumor weight were measured, and pathological analysis of the resected tissue was performed to verify the antitumor effect. The tumor volume was measured from the start of administration until sacrifice by the calculation formula: V = long axis × short axis × short axis/2) (FIGS. 38 and 39).

As a result, in the ANXA1-negative prostate cancer model, no tumor reduction effect was observed in both the EMCS-¹⁰BSH-vc-dhp7 administration and BNCT treatment group and the ¹⁰BSH administration and BNCT treatment group. Thus, it was suggested that boron derived from ¹⁰BSH may accumulate weakly and may not reach a tissue boron concentration sufficient for the antitumor effect of BNCT in ANXA1-negative cancer tissues. Meanwhile, in the ANXA1-positive head and neck cancer model, the EMCS-¹⁰BSH-vc-dhp7 administration and BNCT treatment group exhibited a tumor growth suppressing effect more remarkable than that in the ¹⁰BSH and BNCT treatment group starting immediately after the first BNCT, and exhibited a reduced tumor volume as compared with the ¹⁰BSH and BNCT treatment group at one week after the second irradiation (FIGS. 38 and 39).

The above results suggest that EMCS-¹⁰BSH-vc-dhp7 is a drug more capable of delivering boron to ANXA 1-positive cancer tissues than a conventional drug, because it exhibited an antitumor effect on ANXA1-positive cancers superior to that of ¹⁰BSH alone. ANXA1-positive cancers have been reported in many carcinomas such as head and neck cancer as well as breast cancer, lung cancer, melanoma, brain tumor, renal cancer, and bladder cancer. Conjugates of the peptides of the present invention with boron compounds are believed to be drug candidates useful for BNCT of ANXA 1-positive cancers.

### Example 12: Anticancer agent treatment with c(vcMMAE)-hpnevrs (vcMMAE-dhp7)

Examples 8 to 11 showed that compounds bound to dhp7 via a Val-Cit linker exhibit tumor accumulation and an antitumor effect. Thus, c(vcMMAE)-hpnevrs (vcMMAE-dhp7) was then synthesized (SEQ ID NO: 31 (hpnevrs)), in which a Cys residue is added to the N-terminus of an hpnevrs(dhp7) sequence (SEQ ID NO: 31) and a tubulin inhibitor monomethyl auristatin E (MMAE) is bound thereto via a Val-Cit linker (Formula 7).

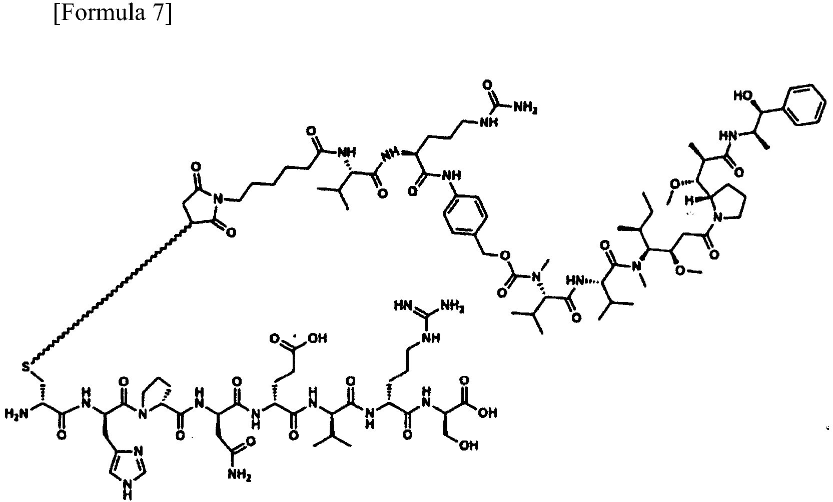

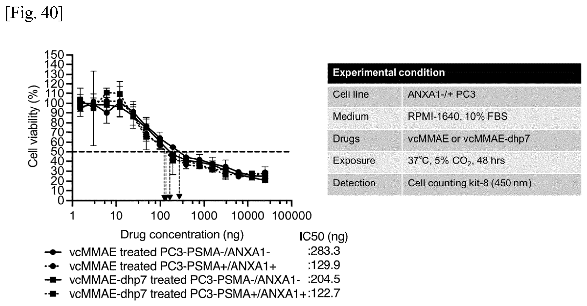

To study cytotoxicity of the synthesized drug to ANXA1-positive/negative PC3 cells in vitro, the peptide-drug conjugate and various concentrations of vcMMAE or vcMMAE-dhp7 were added to the PC3 cells, and the cells were incubated at 37°C for 24 hours. The cell viability was analyzed by Cell Counting Kit-8 (Dojindo), and the IC50 value was calculated (FIG. 40). It was confirmed that the IC50 values of the PC3-Luc2-PSMA+/ANXA1-positive cells treated with vcMMAE or vcMMAE-dhp7 (129.9 ng or 122.7 ng, respectively) were twice those of the PC3-Luc2-PSMA-/ANXA1-negative cells treated with vcMMAE or vcMMAE-dhp7 (283.3 ng or 204.5 ng, respectively). It was suggested that in-vitro cytotoxicity varies depending on whether the PC3 cells express ANXA1. More specifically, it was suggested that vcMMAE-dhp7 exhibits a higher antitumor effect in tumor cells as they express more ANXA1, because the PC3 cells are more sensitive to vcMMAE in vitro as they express more ANXA1.

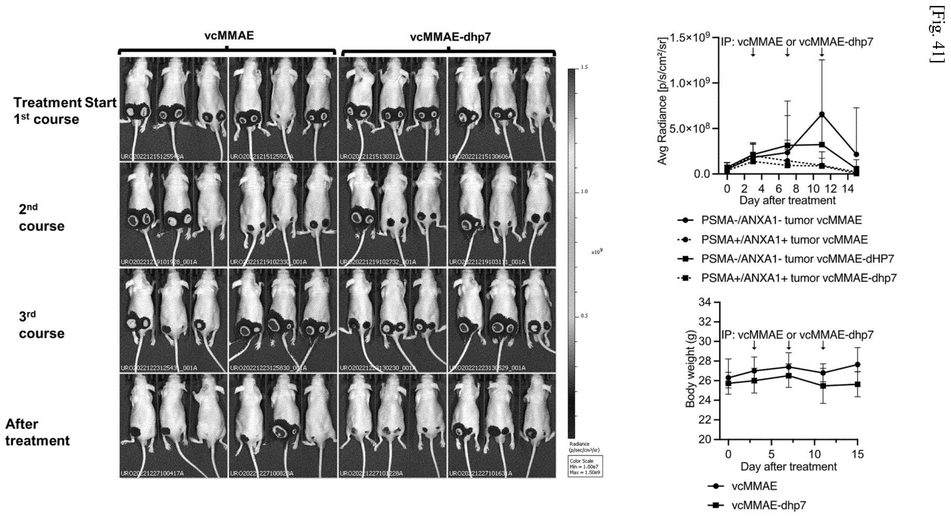

1 × 10⁶ PSMA-negative/ANXA1-negative human prostate cancer cells PC3 were inoculated into the left back of nude mice, and 1 × 10⁶ PSMA-positive/ANXA1-positive PC3 cells were inoculated into the right back of the nude mice. Two weeks later, vcMMAE-dhp7 (molecular weight: 2257.5) dissolved in physiological saline was intraperitoneally administered at 1.25 mg/kg (corresponding to 16.75 ng vcMMAE/25 g mouse) every four days to cancer-bearing mice in which the tumor size reached 7 mm. As control, 0.919 mg/kg (22.975 ng vcMMAE/25 g mouse) of vcMMAE alone (molecular weight: 1316.6) was administered. The tumor size was monitored by measuring the average radiance (photon/sec/cm²/sr) of the luciferase-expressing tumor after injection of luciferin (150 mg/mouse) using an IVIS system. After three courses of administration, the tumor size, body weight, pathological test, blood biochemical test, and blood cell count test values were evaluated (FIG. 41).

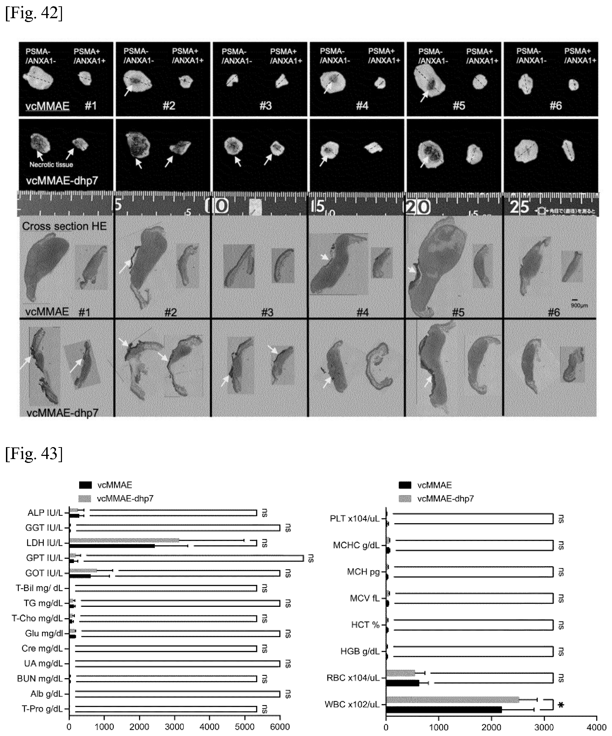

In-vitro sensitivity of the Anxa1-positive cells to vcMMAE and vcMMAE-dhp7 was twice that of the ANXA1-negative cells, resulting in a higher cell-killing effect. Meanwhile, in the mouse model, the average radiance of the PC3 tumor model mice to which vcMMAE-dhp7 was administered was almost completely reduced similarly over the three courses, regardless of the amount of ANXA1 expressed in the tumor (FIG. 41). No remarkable change such as body weight reduction was observed in each group (FIG. 41). FIGS. 42 and 43 show the appearance of resected tumors and the post-treatment blood test results.

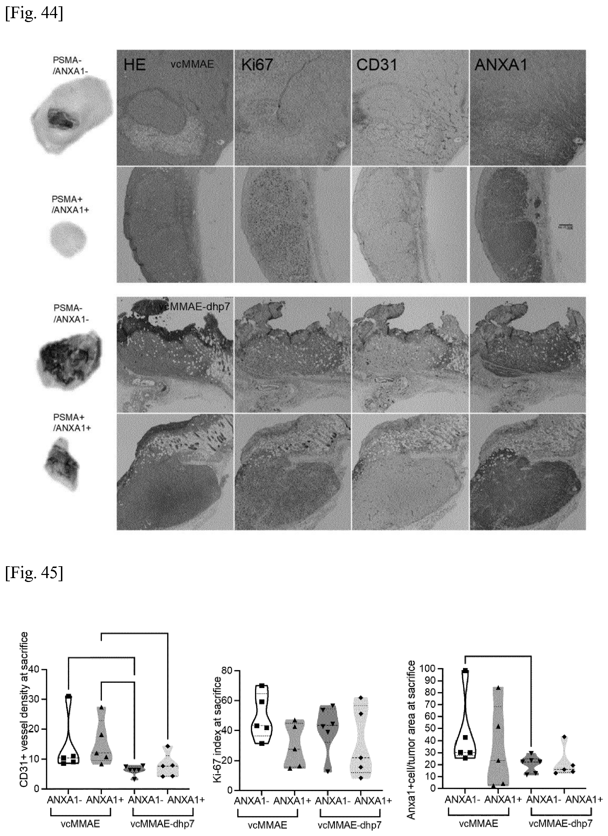

As is clear from the appearance and HE staining results of resected tumors in the vcMMAE-dhp7 administration group, marked necrotic change and fewer residual tumors were observed, in contrast to the vcMMAE alone administration group (FIG. 42). The blood biochemical values in the vcMMAE-dhp7 administration group did not significantly differ from those in the vcMMAE alone administration group, although the white blood cell count was reduced in the vcMMAE alone administration group (FIG. 43). The immunostaining results are shown in FIGS. 44 and 45.

The immunostaining results (FIGS. 44 and 45) showed that there was no statistically significant difference in the Ki67 index and ANXA1 expression between the residual tumors, although the CD31-positive vessel density in the residual tumors in the vcMMAE-dhp7 treatment group was statistically significantly reduced from that in the vcMMAE treatment group. These results show that the vessels in the post-treatment residual tumors were reduced more in the vcMMAE-dhp7 treatment group, regardless of the expression of ANXA1. It is believed that the marked necrotic change in the vcMMAE-dhp7 treatment group is caused not only by the direct antitumor effect of vcMMAE-dhp7 on tumors but also by reduction of tumor nutrient vessels due to the cell-killing effect on the tumor nutrient vessels. The above results showed that vcMMAE-dhp7 can reduce the dose of vcMMAE by about 30% and exhibits an antitumor effect superior to that of vcMMAE.

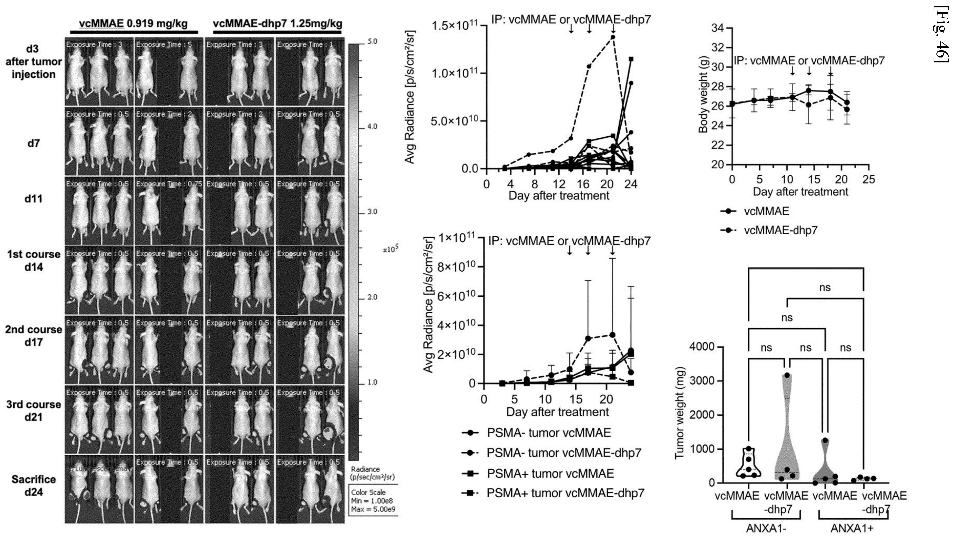

Since the above results showed a superior antitumor effect of vcMMAE-dhp7 in the back subcutaneous tumor model, the same treatment experiment as described above was performed in a tumor model in which cells were subcutaneously inoculated into both femurs. 1 × 10⁶ PSMA-negative/ANXA1-negative human prostate cancer cells PC3 were inoculated into the left femur of nude mice, and 1 × 10⁶ PSMA-positive/ANXA1-positive PC3 cells were inoculated into the right femur of the nude mice. Two weeks later, vcMMAE-dhp7 (molecular weight: 2257.5) dissolved in physiological saline was intraperitoneally administered at 1.25 mg/kg (corresponding to 16.75 ng vcMMAE/25 g mouse) every four days to cancer-bearing mice in which the tumor size reached 7 mm. As control, 0.919 mg/kg (22.975 ng vcMMAE/25 g mouse) of vcMMAE alone (molecular weight: 1316.6) was administered. The tumor size was monitored by measuring the average radiance (photon/sec/cm²/sr) of the luciferase-expressing tumor after injection of luciferin (150 mg/mouse using an IVIS system. After three courses of administration, the average radiance of the luciferase-expressing tumor and the body weight were evaluated (FIG. 46).

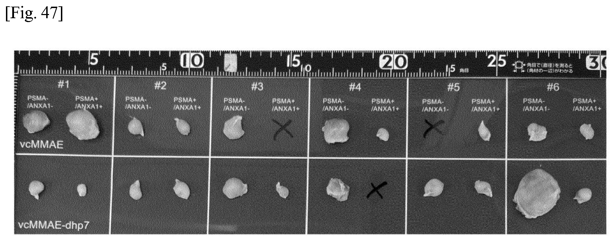

In the model mice in which the PC3 tumor was subcutaneously inoculated into the femur, the average radiance in the vcMMAE-dhp7 administration group was reduced similarly over the three courses of treatment, regardless of the amount of ANXA1 expressed in the tumor and the size of the tumor, while the average radiance in the vcMMAE treatment group tended to increase (FIG. 46). No remarkable change such as body weight reduction was observed in each group (FIG. 46). The appearance of the resected tumors is shown in FIG. 47. The above results showed that vcMMAE-dhp7 can reduce the dose of vcMMAE by about 30% and exhibits an antitumor effect superior to that of vcMMAE.

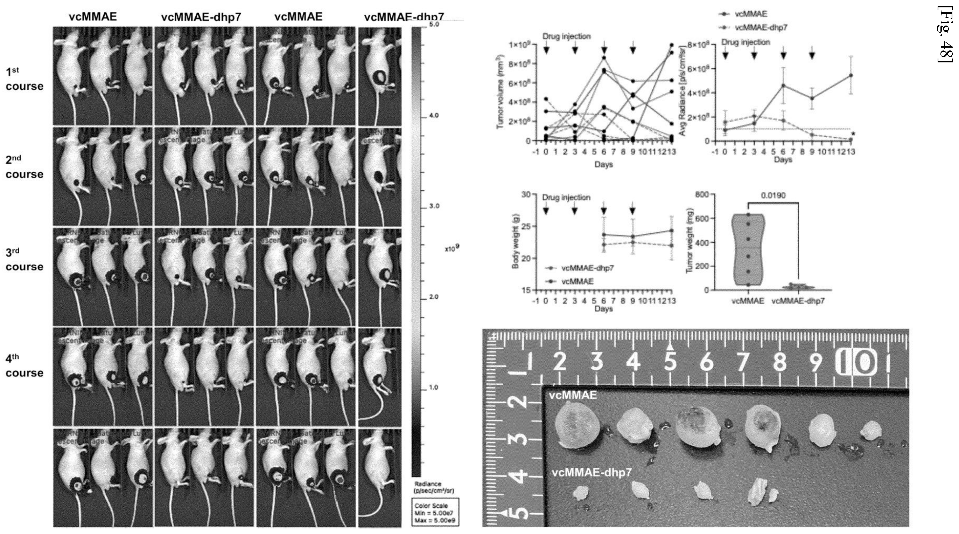

Since the above results showed a superior antitumor effect of vcMMAE-dhp7 in the femur subcutaneous tumor model, the antitumor effect of vcMMAE-dhp7 was then compared with that of the same dose of vcMMAE alone. 2 × 10⁶ PSMA-positive/ANXA1-positive PC3 cells were inoculated into the right femur. One week later, vcMMAE-dhp7 (molecular weight: 2257.5) dissolved in physiological saline was intraperitoneally administered at 2.14 mg/kg (corresponding to 31.25 ng vcMMAE/25 g mouse) every four days to cancer-bearing mice in which the tumor size reached 7 mm. As control, 1.25 mg/kg (31.25 ng vcMMAE/25 g mouse) of vcMMAE alone (molecular weight: 1316.6) was administered. The tumor size was monitored by measuring the average radiance (photon/sec/cm²/sr) of the luciferase-expressing tumor after injection of luciferin (150 mg/mouse using an IVIS system. After four courses of administration, the average radiance of the luciferase-expressing tumor and the body weight were evaluated (FIG. 48).

In the model mice in which the PC3-PSMA-positive/ANXA1-positive tumor was subcutaneously inoculated into the femur, the average radiance in the vcMMAE-dhp7 administration group was markedly reduced over the four courses of treatment with vcMMAE-dhp7, while the average radiance in the vcMMAE treatment group tended to increase (FIG. 48). No remarkable change such as body weight reduction was observed in each group (FIG. 48). The appearance of the resected tumors is shown in FIG. 48. The resected tumor weights in the vcMMAE-dhp7 treatment group were markedly smaller than those in the vcMMAE treatment group. Thus, it was found that vcMMAE-dhp7 can be administered at the same dose as that of vcMMAE, and exhibits an antitumor effect on ANXA1-positive tumors superior to that of vcMMAE.

### Industrial Applicability

According to the present invention, the obtained D-peptide can bind with high affinity to ANXA1 having high specificity among tumor vessel-specific marker molecules and is thus useful. The D-peptide is highly soluble as compared with conventionally known peptides, and is thus extremely useful for PDCs targeting ANXA1. Further, the D-peptide accumulates at the tumor site continuously and remarkably, and can be expected to, for example, improve the efficiency of drug uptake in cancer cells and the antitumor effect in cancer such as bladder cancer, prostate cancer, or head and neck cancer, as compared with conventionally known peptides. Therefore, the D-peptide is extremely useful, for example, for applications such as medical care (e.g., cancer therapy, more specifically, (boron) neutron capture therapy or cancer chemotherapy; or cancer tests, more specifically, monitoring of the therapeutic effect on a disease by in-vivo imaging). In addition, the D-peptide can be expected to reduce side effects of the drug when it is used for a PDC, and is thus useful.

The present application claims the benefit of priority based on Japanese Patent Application No. 2023-137833 filed in Japan on August 28, 2023, the contents of which are incorporated herein in their entirety.

Sequence Listing

## Claims

1. A peptide comprising an amino acid sequence represented by any one of the formulas (I) to (V) (wherein each amino acid symbol immediately following the symbol [D] represents the D-form of the corresponding amino acid):
(I) [D](X1)[D](X2)[D](X3)[D]E[D]V[D]R[D]S, wherein X1 represents H or Q, X2 represents P or S, and X3 represents N or K,
(II) [D]Q[D](X2)[D]A[D]T[D](X5)[D]L[D]K, wherein X2 represents Y or L, and X5 represents N, K, or Y,
(III) [D](X1)[D]T[D]S[D](X4)[D](X5)[D]T[D]L, wherein X1 represents S or R, X4 represents R or W, and X5 represents N or I,
(IV) any one of the amino acid sequences (I) to (III), which has one or several amino acid insertions, one or several amino acid substitutions, or one or several amino acid deletions, or a combination thereof, and
(V) an amino acid sequence that is a retro-inverso of any one of the amino acid sequences (I) to (IV).

2. The peptide according to claim 1, comprising any one of the amino acid sequences (I') to (V) (wherein the symbol [D] is as defined above):
(I') [D](X1)[D](X2)[D]N[D]E[D]V[D]R[D]S, wherein X1 represents H or Q, and X2 represents P or S,
(II) [D]Q[D](X2)[D]A[D]T[D](X5)[D]L[D]K, wherein X2 represents Y or L, and X5 represents N, K, or Y,
(III) [D](X1)[D]T[D]S[D](X4)[D](X5)[D]T[D]L, wherein X1 represents S or R, X4 represents R or W, and X5 represents N or I,
(IV) any one of the amino acid sequences (I') to (III), which has one or several amino acid insertions, one or several amino acid substitutions, or one or several amino acid deletions, or a combination thereof, and
(V) an amino acid sequence that is a retro-inverso of any one of the amino acid sequences (I') to (IV).

3. The peptide according to claim 1 or 2, comprising any one of the amino acid sequences (i) to (iii) (wherein the symbol [D] is as defined above):
(i) [D]H[D]P[D]N[D]E[D]V[D]R[D]S,
(ii) [D]S[D]T[D]S[D]R[D]N[D]T[D]L, and
(iii) [D]Q[D]Y[D]A[D]T[D]N[D]L[D]K.

4. The peptide according to any one of claims 1 to 3, comprising an amino acid sequence represented by the formula (i) (wherein the symbol [D] is as defined above):
(i) [D]H[D]P[D]N[D]E[D]V[D]R[D]S.

5. A conjugate comprising the peptide according to any one of claims 1 to 4 and one or more components.

6. The conjugate according to claim 5, wherein the one or more components include a boron or gadolinium compound.

7. The conjugate according to claim 6, wherein the one or more components include a boron compound.

8. The conjugate according to claim 7, wherein the conjugate is represented by the formula (I): 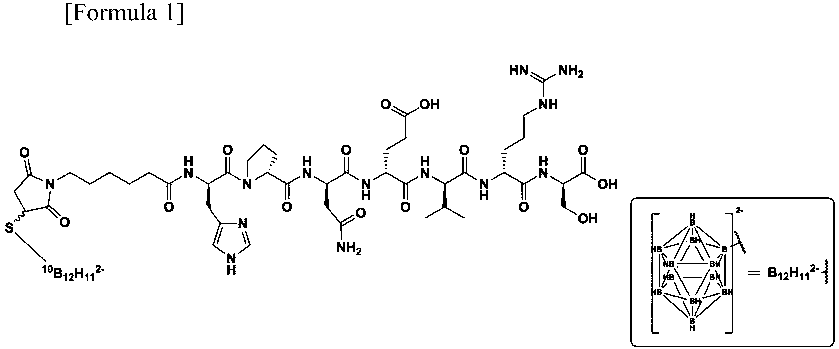 or the formula (II):

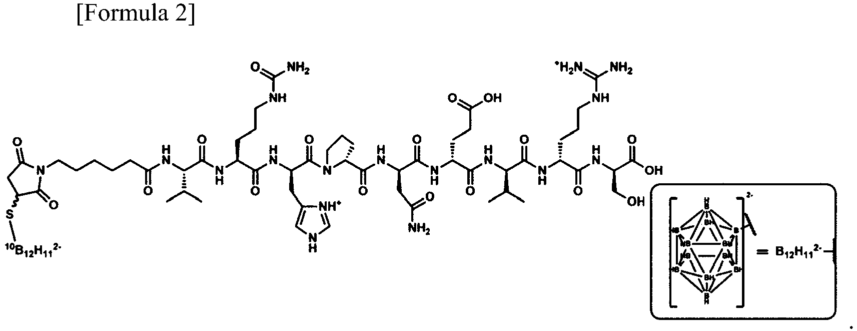

9. The conjugate according to claim 5, wherein the one or more components include an anticancer agent.

10. The conjugate according to claim 5, wherein the one or more components include a detectable substance.

11. The conjugate according to claim 10, wherein the detectable substance allows the conjugate to be detected in vivo by a means selected from the group consisting of radiography, computed tomography (CT), magnetic resonance imaging (MRI), ultrasonography, scintigraphy, positron emission tomography (PET), radionuclide therapy, endoscopy, and laparoscopy.

12. The conjugate according to claim 10 or 11, wherein the detectable substance is a radioisotope, an MRI contrast enhancer, a radiopaque substance, a contrast medium, or a fluorescent substance.

13. A pharmaceutical composition comprising the peptide according to any one of claims 1 to 4 or the conjugate according to any one of claims 5 to 12.

14. A neutron capture therapy agent comprising the conjugate according to any one of claims 6 to 8.

15. The neutron capture therapy agent according to claim 14, wherein the conjugate includes a boron compound.

16. The neutron capture therapy agent according to claim 14 or 15, for the treatment or prevention of solid cancer.

17. The neutron capture therapy agent according to claim 16, wherein the solid cancer is annexin A1-positive solid cancer.

18. A cancer chemotherapeutic agent comprising the conjugate according to claim 9.

19. A cancer testing reagent comprising the conjugate according to any one of claims 10 to 12.
